# EUROPEAN PATENT SPECIFICATION

(11) **EP 1 849 868 B1**
(45) Date of publication and mention of the grant of the patent: **02.12.2009**
(21) Application number: 07011828.6
(22) Date of filing: 03.05.2000
(51) Int. Cl.: C12N 15/29, C12N 15/82, C07K 14/415, C12N 5/10, C12Q 1/68, A01H 5/00

(54) **Plant defensins**
Pflanzliche Defensine
Défensines végétales

(30) Priority: 07.05.1999 US 133039 P
(43) Date of publication of application: 31.10.2007
(62) Divisional of application: 00928736.8
(73) Proprietor: E.I. DU PONT DE NEMOURS AND COMPANY, Wilmington, DE 19898 (US)
(72) Inventor: Weng, Zude, Vernon Hills, IL 60061 (US); Miao, Guo-Hua, Hockessin, DE 19707 (US); Famodu, Omolayo O., Newark, DE 19702 (US)
(74) Representative: Bentham, Andrew

(56) References cited:
- WO-A-00/11196
- WO-A-93/05153
- WO-A-94/16076
- WO-A-97/21814
- WO-A-97/21815
- WO-A-97/37024

## Description

### FIELD OF THE INVENTION

This invention is in the field of plant molecular biology. More specifically, this invention pertains to nucleic acid fragments encoding plant defensins.

### BACKGROUND OF THE INVENTION

Defensins are small, basic, cysteine-rich proteins that have broad antimicrobial activity through the formation of multimeric pores in outer or inner biological membranes that lead to membrane disruption and depolarization. They have a wide phylogenetic distribution, having been found in insects, mammals, and plants.

Although plant defensins have only been identified recently and are not as well-characterized as their mammalian and insect counterparts, several lines of observation suggest the importance of defensins in mediating host resistance to microbial attack. Plant defensins have been shown to induce a rapid K+ efflux and Ca2+ influx in fungal hyphae as well as alkalinization of the incubation medium. The operating mechanism however appears not to involve direct defensin-membrane interactions, but rather a different, possibly receptor-mediated event (Thevissen, K. et al. (1996) J. Biol. Chem. 271:15018-15025). Defensins have also been shown to accumulate systemically upon challenge by fungal pathogens (Manners, J. M. et al. (1998) Plant Mol. Biol. 38:1071-1080; Terras, F. R. et al. (1998) Planta 206:117-124; Terras, F. R. et al. (1995) Plant Cell 7:573-588). Furthermore, transgenic tobacco that expressed constitutively a radish defensin was found to have improved resistance to infection by a fungal pathogen (Terras, F. R et al., (1995) Plant Cell 7:573-588).

Defensins have been shown to be induced by artificial drought (Maitra, N. and Cushman, J. C., (1998) Plant Physiol. 118:1536) and salt stress (Yamada, S. et al. (1997) Plant Physiol. 115:314), suggesting that these proteins may play a more general role in stress tolerance, a role that is not restriced to pathogen attack. Plant defensins with antifungal and anti-bacterial activity have also been shown in WO 9305153.

Isolation of additional plant defensin genes may lead to a better understanding of the mechanism of action of defensin. It is possible that with these genes in hand, defensins with increased cytotoxicity and pathogen specificity may be designed, and transgenic plants with increased disease resistance and stress tolerance may be generated.

### SUMMARY OF THE INVENTION

The invention provides an isolated polynucleotide encoding a defensin polypeptide having antimicrobial activity and comprising a nucleotide sequence selected from:
(a) a nucleotide sequence encoding a polypeptide of at least 49 amino acids that has at least 95% identity based on the Clustal method of alignment when compared to a polypeptide of SEQ ID NO:8; and
(b) a nucleotide sequence encoding a polypeptide of at least 49 amino acids that has at least 90% identity based on the Clustal method of alignment when compared to a polypeptide of SEQ ID NO: 6.

The nucleotide sequences may be DNA or RNA.

The invention also provides a chimeric gene comprising the isolated polynucleotide of the invention operably linked to at least one suitable regulatory sequence.

The invention also provides a host cell comprising the chimeric gene of the invention.

The invention also provides a host cell comprising the isolated polynucleotide of the invention.

The invention also provides a virus comprising the isolated polynucleotide of the invention.

The invention also provides a defensin polypeptide having antimicrobial activity and selected from:
(a) a polypeptide of at least 49 amino acids that has at least 90% identity based on the Clustal method of alignment when compared to a polypeptide of SEQ ID NO: 6; and
(b) a polypeptide of at least 50 amino acids that has at least 95% identity based on the Clustal method of alignment when compared to a polypeptide of SEQ ID NO: 8.

The invention also provides a composition comprising the isolated polynucleotide of the invention.

The invention also provides a composition comprising the isolated polypeptide of the invention.

The invention also provides a method for positive selection of a transformed cell comprising:
(a) transforming a host cell with the chimeric gene of the invention; and
(b) growing the transformed host cell under conditions which allow expression of a polynucleotide in an amount sufficient to complement a null mutant to provide a positive selection means.

The invention also provides a plant transformed with a polynucleotide of the invention such as a polypeptide having antimicrobial activity is expressed to a level that exceeds the level of production in a non-transformed plant.

The invention also provides a method of producing the plant of the invention comprising stably transferring a polynucleotide of the invention into the genome of a plant host cell and establishing a transgenic plant therefrom.

### BRIEF DESCRIPTION OF THE DRAWING AND SEQUENCE LISTINGS

The invention can be more fully understood from the following detailed description and the accompanying drawing and Sequence Listing which form a part of this application.

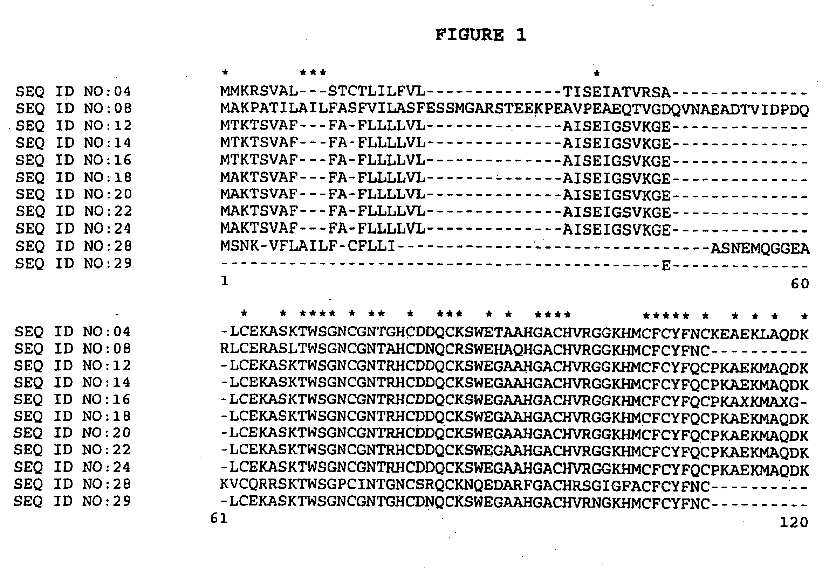

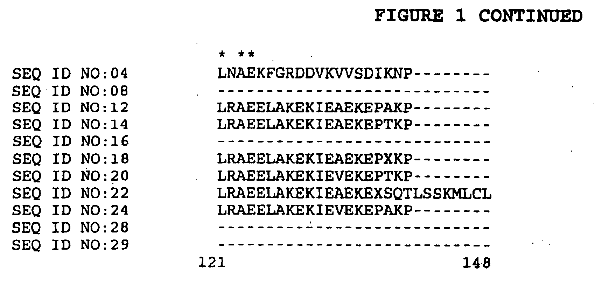

Figure 1 depicts the amino acid sequence alignment between the defensin encoded by the nucleotide sequences derived from *Dimorphotheca sinuata* clone dms2c.pk001.d3 (SEQ ID NO:4), *Picramnia pentandra* clone pps.pk0011.a9 (SEQ ID NO: 8), *Parthenium argentatum* Grey clones epb1c.pk002.h2 (SEQ ID NO: 12), epblc.pk001.h15 (SEQ ID NO:14), epb1c.pk003.p14 (SEQ ID NO:16), epb1c.pk004.p22 (SEQ ID NO:18), epbl c.pkOO5.o6 (SEQ ID NO:20), epblc.pk006.k15 (SEQ ID NO:22), and epb3c.pk009.j22 (SEQ ID NO:24), and *Nicotiana benthamiana* clone tdrlc.pk002.g7 (SEQ ID NO:28) and the defensin polypeptide isolated from *Dahlia merckii* (NCBI GenBank Identifier (GI) No. 2147320; SEQ ID NO:29). Amino acids which are conserved among all and at least two sequences with an amino acid at that position are indicated with an asterisk (*). Dashes are used by the program to maximize alignment of the sequences.

Table 1 lists the polypeptides that are described herein, the designation of the cDNA clones that comprise the nucleic acid fragments encoding polypeptides representing all or a substantial portion of these polypeptides, and the corresponding identifier (SEQ ID NO:) as used in the attached Sequence Listing. Table 1 also identifies the cDNA clones as individual ESTs ("EST"), the sequences of the entire cDNA inserts comprising the indicated cDNA clones ("FIS"), contigs assembled from two or more ESTs ("Contig"), contigs assembled from an FIS and one or more ESTs ("Contig*"), or sequences encoding at a minimum the mature protein derived from an EST, FIS, a contig, or an FIS and PCR ("CGS"). Nucleotide SEQ ID NOs:1, 5, 9, and 25 correspond to nucleotide SEQ ID NOs:1, 5, 3, and 7, respectively, presented in U.S. Provisional Application No. 60/133039. filed May 7, 1999. Amino acid SEQ ID NOs:2, 6, 10, and 26 correspond to amino acid SEQ ID NOs:2, 6, 4, and 8, respectively, presented in U.S. Provisional Application No. 60/133,039, filed May 7, 1999.

**TABLE I**

| Plant Defensins | | | | |
|---|---|---|---|---|
| | | | SEQ ID NO: | |
| Protein (Plant Source) | Clone Designation | Status | (Nucleotide) | (Amino Acid) |
| Plant Defensin | dms2c.pk001.d3 | EST | 1 | 2 |
| (*Dimorphotheca sinuata*) | | | | |
| Plant Defensin | dms2c.pk001.d3(FIS) | CGS | 3 | 4 |
| *(Dimorphotheca sinuata)* | | | | |
| Plant Defensin | pps.pk001 1:a9 | EST | 5 | 6 |
| *(Picramnia pentandra)* | | | | |
| Plant Defensin | pps.pk0011.a9(FIS) | CGS | 7 | 8 |
| *(Picramnia pentandra)* | | | | |
| Plant Defensin | epblc.pk002.h2(EST) | CGS | 9 | 10 |
| *(Parthenium argentatum* Grey) | | | | |
| Plant Defensin | epblc.pk002.h2(FIS) | CGS | 11 | 12 |
| *(Parthenium argentatum* Grey) | | | | |
| Plant Defensin | epblc.pk001.h15(EST) | CGS | 13 | 14 |
| *(Parthenium argentatum* Grey) | | | | |
| Plant Defensin | epblc.pk003.p14(EST) | CGS | 15 | 16 |
| (*Parthenium argentatum* Grey) | | | | |
| Plant Defensin | epblc.pk004.p22(EST) | CGS | 17 | 18 |
| (*Parthenium argentatum* Grey) | | | | |
| Plant Defensin | epblc.pk005.o6(EST) | CGS | 19 | 20 |
| (*Parthenium argentatum* Grey) | | | | |
| Plant Defensin | epblc.pk006.k15(EST) | CGS | 21 | 22 |
| (*Parthenium argentatum* Grey) | | | | |
| Plant Defensin | epb3c.pk009.j22(EST) | CGS | 23 | 24 |
| *(Parthenium argentatum* Grey) | | | | |
| Plant Defensin (*Nicotiana benthamiana)* | tdrlc.pk002.g7(EST) | CGS | 25 | 26 |
| Plant Defensin *(Nicotiana benthamiana)* | tdrlc.pk002.g7(FIS) | CGS | 27 | 28 |

The Sequence Listing contains the one letter code for nucleotide sequence characters and the three letter codes for amino acids as defined in conformity with the IUPAC-IUBMB standards described in Nucleic Acids Res. 13:3021-3030 (1985) and in the Biochemical J. 219 (No. 2):345-373 (1984)

### DETAILED DESCRIPTION OF THE INVENTION

In the context of this disclosure, a number of terms shall be utilized. The terms "polynucleotide", "polynucleotide sequence", "nucleic acid sequence", and "nucleic acid fragment"/"isolated nucleic acid fragment" are used interchangeably herein. These terms encompass nucleotide sequences and the like. A polynucleotide may be a polymer of RNA or DNA that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof.

The term "isolated polynucleotide" refers to a polynucleotide that is substantially free from other nucleic acid sequences, such as and not limited to other chromosomal and extrachromosomal DNA and RNA. Isolated polynucleotides may be purified from a host cell in which they naturally occur. Conventional nucleic acid purification methods known to skilled artisans may be used to obtain isolated polynucleotides. The term also embraces recombinant polynucleotides and chemically synthesized polynucleotides.

The term "recombinant" means, for example, that a nucleic acid sequence is made by an artificial combination of two otherwise separated segments of sequence, e.g., by chemical synthesis or by the manipulation of isolated nucleic acids by genetic engineering techniques.

As used herein, "substantially similar" refers to nucleic acid fragments wherein changes in one or more nucleotide bases results in substitution of one or more amino acids, but do not affect the functional properties of the polypeptide encoded by the nucleotide sequence. "Substantially similar" also refers to nucleic acid fragments wherein changes in one or more nucleotide bases does not affect the ability of the nucleic acid fragment to mediate alteration of gene expression by gene silencing through for example antisense or co-suppression technology. "Substantially similar" also refers to modifications of the nucleic acid fragments of the instant invention such as deletion or insertion of one or more nucleotides that do not substantially affect the functional properties of the resulting transcript vis-à-vis the ability to mediate gene silencing or alteration of the functional properties of the resulting protein molecule. It is therefore understood that the invention encompasses more than the specific exemplary nucleotide or amino acid sequences and includes functional equivalents thereof. The terms "substantially similar" and "corresponding substantially" are used interchangeably herein.

Substantially similar nucleic acid fragments may be selected by screening nucleic acid fragments representing subfragments or modifications of the nucleic acid fragments of the instant invention, wherein one or more nucleotides are substituted, deleted and/or inserted, for their ability to affect the level of the polypeptide encoded by the unmodified nucleic acid fragment in a plant or plant cell. For example, a substantially similar nucleic acid fragment representing at least one of 30 contiguous nucleotides derived from the instant nucleic acid fragment can be constructed and introduced into a plant or plant cell. The level of the polypeptide encoded by the unmodified nucleic acid fragment present in a plant or plant cell exposed to the substantially similar nucleic fragment can then be compared to the level of the polypeptide in a plant or plant cell that is not exposed to the substantially similar nucleic acid fragment.

For example, it is well known in the art that antisense suppression and co-suppression of gene expression may be accomplished using nucleic acid fragments representing less than the entire coding region of a gene, and by using nucleic acid fragments that do not share 100% sequence identity with the gene to be suppressed. Moreover, alterations in a nucleic acid fragment which result in the production of a chemically equivalent amino acid at a given site, but do not effect the functional properties of the encoded polypeptide, are well known in the art. Thus, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a functionally equivalent product. Nucleotide changes which result in alteration of the N-terminal and C-terminal portions of the polypeptide molecule would also not be expected to alter the activity of the polypeptide. Each of the proposed modifications is well within the routine skill in the art, as is determination of retention of biological activity of the encoded products. nucleotide sequence of one invention may be used in methods of selecting an isolated polynucleotide that affects the expression of a plant defensin polypeptide in a host cell. A method of selecting an isolated polynucleotide that affects the level of expression of a polypeptide in a virus or in a host cell (eukaryotic, such as plant or yeast, prokaryotic such as bacterial) may comprise the steps of: constructing an isolated polynucleotide of the present invention or a chimeric gene of the present invention; introducing the isolated polynucleotide or the chimeric gene into a host cell; measuring the level of a polypeptide or enzyme activity in the host cell containing the isolated polynucleotide; and comparing the level of a polypeptide or enzyme activity in the host cell containing the isolated polynucleotide with the level of a polypeptide or enzyme activity in a host cell that does not contain the isolated polynucleotide.

Moreover, substantially similar nucleic acid fragments may also be characterized by their ability to hybridize. Estimates of such homology are provided by either DNA-DNA or DNA-RNA hybridization under conditions of stringency as is well understood by those skilled in the art (Hames and Higgins, Eds. (1985) Nucleic Acid Hybridisation, IRL Press, Oxford, U.K.). Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions. One set of preferred conditions uses a series of washes starting with 6X SSC, 0.5% SDS at room temperature for 15 min, then repeated with 2X SSC, 0.5% SDS at 45°C for 30 min, and then repeated twice with 0.2X SSC, 0.5% SDS at 50°C for 30 min. A more preferred set of stringent conditions uses higher temperatures in which the washes are identical to those above except for the temperature of the final two 30 min washes in 0.2X SSC, 0.5% SDS was increased to 60°C. Another preferred set of highly stringent conditions uses two final washes in 0.1X SSC, 0.1% SDS at 65°C.

Substantially similar nucleic acid fragments of the instant invention may also be characterized by the percent identity of the amino acid sequences that they encode to the amino acid sequences disclosed herein, as determined by algorithms commonly employed by those skilled in this art. Suitable nucleic acid fragments (isolated polynucleotides of the present invention) encode polypeptides that are at least about 70% identical, preferably at least about 80% identical to the amino acid sequences reported herein. Preferred nucleic acid fragments encode amino acid sequences that are about 85% identical to the amino acid sequences reported herein. More preferred nucleic acid fragments encode amino acid sequences that are at least about 90% identical to the amino acid sequences reported herein. Most preferred are nucleic acid fragments that encode amino acid sequences that are at least about 95% identical to the amino acid sequences reported herein. Suitable nucleic acid fragments not only have the above identities but typically encode a polypeptide having at least 49 or 50 amino acids, preferably at least 100 amino acids, more preferably at least 150 amino acids, still more preferably at least 200 amino acids, and most preferably at least 250 amino acids. Sequence alignments and percent identity calculations were performed using the Megalign program of the LASERGENE bioinformatics computing suite (DNASTAR Inc., Madison, WI). Multiple alignment of the sequences was performed using the Clustal method of alignment (Higgins and Sharp (1989) CABIOS. 5:151-153) with the default parameters (GAP PENALTY=10, GAP LENGTH PENALTY=10). Default parameters for pairwise alignments using the Clustal method were KTUPLE 1, GAP PENALTY=3, WINDOW=5 and DIAGONALS SAVED=5.

A "substantial portion" of an amino acid or nucleotide sequence comprises an amino acid or a nucleotide sequence that is sufficient to afford putative identification of the-protein or gene that the amino acid or nucleotide sequence comprises. Amino acid and nucleotide sequences can be evaluated either manually by one skilled in the art, or by using computer-based sequence comparison and identification tools that employ algorithms such as BLAST (Basic Local Alignment Search Tool; Altschul et al. (1993) J. Mol. Biol. 215:403-410; see also www.ncbi.nlm.nih.gov/BLAST/). In general, a sequence of ten or more contiguous amino acids or thirty or more contiguous nucleotides is necessary in order to putatively identify a polypeptide or nucleic acid sequence as homologous to a known protein or gene. Moreover, with respect to nucleotide sequences, gene-specific oligonucleotide probes comprising 30 or more contiguous nucleotides may be used in sequence-dependent methods of gene identification (e.g., Southern hybridization) and isolation (e.g.*, in situ* hybridization of bacterial colonies or bacteriophage plaques). In addition, short oligonucleotides of 12 or more nucleotides may be used as amplification primers in PCR in order to obtain a particular nucleic acid fragment comprising the primers. Accordingly, a "substantial portion" of a nucleotide sequence comprises a nucleotide sequence that will afford specific identification and/or isolation of a nucleic acid fragment comprising the sequence. The instant specification teaches amino acid and nucleotide sequences encoding polypeptides that comprise one or more particular plant proteins. The skilled artisan, having the benefit of the sequences as reported herein, may now use all or a substantial portion of the disclosed sequences for purposes known to those skilled in this art. Accordingly, the instant invention comprises the complete sequences as reported in the accompanying Sequence Listing, as well as substantial portions of those sequences as defined above.

"Codon degeneracy" refers to divergence in the genetic code permitting variation of the nucleotide sequence without effecting the amino acid sequence of an encoded polypeptide. Accordingly, the instant invention relates to any nucleic acid fragment comprising a nucleotide sequence that encodes all or a substantial portion of the amino acid sequences set forth herein. The skilled artisan is well aware of the "codon-bias" exhibited by a specific host cell in usage of nucleotide codons to specify a given amino acid. Therefore, when synthesizing a nucleic acid fragment for improved expression in a host cell, it is desirable to design the nucleic acid fragment such that its frequency of codon usage approaches the frequency of preferred codon usage of the host cell.

"Synthetic nucleic acid fragments" can be assembled from oligonucleotide building blocks that are chemically synthesized using procedures known to those skilled in the art. These building blocks are ligated and annealed to form larger nucleic acid fragments which may then be enzymatically assembled to construct the entire desired nucleic acid fragment. "Chemically synthesized", as related to a nucleic acid fragment, means that the component nucleotides were assembled *in vitro.* Manual chemical synthesis of nucleic acid fragments may be accomplished using well established procedures, or automated chemical synthesis can be performed using one of a number of commercially available machines. Accordingly, the nucleic acid fragments can be tailored for optimal gene expression based on optimization of the nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell where sequence information is available.

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign gene" refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

"Coding sequence" refers to a nucleotide sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, and polyadenylation recognition sequences.

"Promoter" refers to a nucleotide sequence capable of controlling the expression of a coding sequence or functional RNA. In general, a coding sequence is located 3' to a promoter sequence. The promoter sequence consists of proximal and more distal upstream elements, the latter elements often referred to as enhancers. Accordingly, an "enhancer" is a nucleotide sequence which can stimulate promoter activity and may be an innate element of the promoter or a heterologous element inserted to enhance the level or tissue-specificity of a promoter. Promoters may be derived in their entirety from a native gene, or may be composed of different elements derived from different promoters found in nature, or may even comprise synthetic nucleotide segments. It is understood by those skilled in the art that different promoters may direct the expression of a gene in different tissues or cell types, or at different stages of development, or in response to different environmental conditions. Promoters which cause a nucleic acid fragment to be expressed in most cell types at most times are commonly referred to as "constitutive promoters". New promoters of various types useful in plant cells are constantly being discovered; numerous examples may be found in the compilation by Okamuro and Goldberg (1989) Biochemistry of Plants 15:1-82*.* It is further recognized that since in most cases the exact boundaries of regulatory sequences have not been completely defined, nucleic acid fragments of different lengths may have identical promoter activity.

"Translation leader sequence" refers to a nucleotide sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner and Foster (1995) Mol. Biotechnol. 3:225-236).

"3' non-coding sequences" refers to nucleotide sequences located downstream of a coding sequence and includes polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al. (1989) Plant Cell 1:671-680.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from posttranscriptional processing of the primary transcript and is referred to as the mature RNA. "Messenger RNA (mRNA)" refers to the RNA that is without introns and that can be translated into polypeptides by the cell. "cDNA" refers to DNA that is complementary to and derived from an mRNA template. The cDNA can be single-stranded or converted to double stranded form using, for example, the Klenow fragment of DNA polymerase I. "Sense RNA" refers to an RNA transcript that includes the mRNA and can be translated into a polypeptide by the cell. "Antisense RNA" refers to an RNA transcript that is complementary to all or part of a target primary transcript or mRNA and that blocks the expression of a target gene (see U.S. Patent No. 5,107,065, incorporated herein by reference). The complementarity of an antisense RNA may be with any part of the specific nucleotide sequence, i.e., at the 5' non-coding sequence, 3' non-coding sequence, introns, or the coding sequence. "Functional RNA" refers to sense RNA, antisense RNA, ribozyme RNA, or other RNA that may not be translated but yet has an effect on cellular processes.

The term "operably linked" refers to the association of two or more nucleic acid fragments so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

The term "expression", as used herein, refers to the transcription and stable accumulation of sense (mRNA) or antisense RNA derived from the nucleic acid fragment of the invention. "Expression" may also refer to translation of mRNA into a polypeptide. "Antisense inhibition" refers to the production of antisense RNA transcripts capable of suppressing the expression of the target protein. "Overexpression" refers to the production of a gene product in transgenic non-human organisms that exceeds levels of production in normal or non-transformed organisms. "Underexpression" refers to the production of a gene product in transgenic non-human organisms at levels below that of levels of production in normal or non-transformed organisms. "Co-suppression" refers to the production of sense RNA transcripts capable of suppressing the expression of identical or substantially similar foreign or endogenous genes (U.S. Patent No. 5,231,020).

A "protein" or "polypeptide" is a chain of amino acids arranged in a specific order determined by the coding sequence in a polynucleotide encoding the polypeptide. Each protein or polypeptide has a unique function.

"Altered levels" or "altered expression" refers to the production of gene product(s) in transgenic non-human organisms in amounts or proportions that differ from that of normal or non-transformed organisms.

"Null mutant" refers to a host cell which either lacks the expression of a certain polypeptide or expresses a polypeptide which is inactive or does not have any detectable expected enzymatic function.

"Mature protein" refers to a post-translationally processed polypeptide; i.e., one from which any pre- or propeptides present in the primary translation product have been removed. "Precursor protein" refers to the primary product of translation of mRNA; i.e., with pre- and propeptides still present. Pre- and propeptides may be but are not limited to intracellular localization signals.

A "chloroplast transit peptide" is an amino acid sequence which is translated in conjunction with a protein and directs the protein to the chloroplast or other plastid types present in the cell in which the protein is made. "Chloroplast transit sequence" refers to a nucleotide sequence that encodes a chloroplast transit peptide. A "signal peptide" is an amino acid sequence which is translated in conjunction with a protein and directs the protein to the secretory system (Chrispeels (1991) Ann. Rev. Plant Phys. Plant Mol. Biol. 42:21-53). If the protein is to be directed to a vacuole, a vacuolar targeting signal (*supra*) can further be added, or if to the endoplasmic reticulum, an endoplasmic reticulum retention signal *(supra)* may be added. If the protein is to be directed to the nucleus, any signal peptide present should be removed and instead a nuclear localization signal included (Raikhel (1992) Plant Phys. 100:1627-1632)*.*

"Transformation" refers to the transfer of a nucleic acid fragment into the genome of a host non-human organism, resulting in genetically stable inheritance. Host non-human organisms containing the transformed nucleic acid fragments are referred to as "transgenic" organisms. Examples of methods of plant transformation include *Agrobacterium*-mediated transformation (De Blaere et al. (1987) Meth. Enzymol. 143:277) and particle-accelerated or "gene gun" transformation technology (Klein et al. (1987) Nature (London) 327:70-73; U.S. Patent No. 4,945,050, incorporated herein by reference). Thus, isolated polynucleotides of the present invention can be incorporated into recombinant constructs, typically DNA constructs, capable of introduction into and replication in a host cell. Such a construct can be a vector that includes a replication system and sequences that are capable of transcription and translation of a polypeptide-encoding sequence in a given host cell. A number of vectors suitable for stable transfection of plant cells or for the establishment of transgenic plants have been described in, e.g., Pouwels et al., Cloning Vectors: A Laboratory Manual, 1985, supp. 1987; Weissbach and Weissbach, Methods for Plant Molecular Biology, Academic Press, 1989; and Flevin et al., Plant Molecular Biology Manual, Kluwer Academic Publishers, 1990. Typically, plant expression vectors include, for example, one or more cloned plant genes under the transcriptional control of 5' and 3' regulatory sequences and a dominant selectable marker. Such plant expression vectors also can contain a promoter regulatory region (e.g., a regulatory region controlling inducible or constitutive, environmentally- or developmentally-regulated, or cell- or tissue-specific expression), a transcription initiation start site, a ribosome binding site, an RNA processing signal, a transcription termination site, and/or a polyadenylation signal.

Standard recombinant DNA and molecular cloning techniques used herein are well known in the art and are described more fully in Sambrook et al. Molecular Cloning: A Laboratory Manual; Cold Spring Harbor Laboratory Press: Cold Spring Harbor, 1989 (hereinafter "Maniatis").

"PCR" or "polymerase chain reaction" is a technique used for the amplification of specific DNA segments (U.S. Patent Nos. 4,683,195 and 4,800,159).

Preferably, the first nucleotide sequence comprises a nucleic acid sequence of SEQ ID NOs:5 or 7 that codes for the polypeptide of SEQ ID NOs:6 or 8.

Nucleic acid fragments encoding at least a substantial portion of several plant defensins have been isolated and identified by comparison of random plant cDNA sequences to public databases containing nucleotide and protein sequences using the BLAST algorithms well known to those skilled in the art. The nucleic acid fragments of the instant invention may be used to isolate cDNAs and genes encoding homologous proteins from the same or other plant species. Isolation of homologous genes using sequence-dependent protocols is well known in the art. Examples of sequence-dependent protocols include, but are not limited to, methods of nucleic acid hybridization, and methods of DNA and RNA amplification as exemplified by various uses of nucleic acid amplification technologies (e.g., polymerase chain reaction, ligase chain reaction).

For example, genes encoding other plant defensin, either as cDNAs or genomic DNAs, could be isolated directly by using all or a substantial portion of the instant nucleic acid fragments as DNA hybridization probes to screen libraries from any desired plant employing methodology well known to those skilled in the art. Specific oligonucleotide probes based upon the instant nucleic acid sequences can be designed and synthesized by methods known in the art (Maniatis). Moreover, the entire sequence(s) can be used directly to synthesize DNA probes by methods known to the skilled artisan such as random primer DNA labeling nick translation, end-labeling techniques, or RNA probes using available *in vitro* transcription systems. In addition, specific primers can be designed and used to amplify a part or all of the instant sequences. The resulting amplification products can be labeled directly during amplification reactions or labeled after amplification reactions, and used as probes to isolate full length cDNA or genomic fragments under conditions of appropriate stringency.

In addition, two short segments of the instant nucleic acid fragments may be used in polymerase chain reaction protocols to amplify longer nucleic acid fragments encoding homologous genes from DNA or RNA. The polymerase chain reaction may also be performed on a library of cloned nucleic acid fragments wherein the sequence of one primer is derived from the instant nucleic acid fragments, and the sequence of the other primer takes advantage of the presence of the polyadenylic acid tracts to the 3' end of the mRNA precursor encoding plant genes. Alternatively, the second primer sequence may be based upon sequences derived from the cloning vector. For example, the skilled artisan can follow the RACE protocol (Frohman et al. (1988) Proc. Natl. Acad. Sci. USA 85:8998-9002) to generate cDNAs by using PCR to amplify copies of the region between a single point in the transcript and the 3' or 5' end. Primers oriented in the 3' and 5' directions can be designed from the instant sequences. Using commercially available 3' RACE or 5' RACE systems (BRL), specific 3' or 5' cDNA fragments can be isolated (Ohara et al. (1989) Proc. Natl. Acad. Sci. USA 86:5673-5677; Loh et al. (1989) Science 243:217-220). Products generated by the 3' and 5' RACE procedures can be combined to generate full-length cDNAs (Frohman and Martin (1989) Techniques 1:165). Consequently, a polynucleotide comprising a nucleotide sequence of at least one of 60 (preferably one of at least 40, most preferably one of at least 30) contiguous nucleotides derived from a nucleotide sequence of the invention and the complement of such nucleotide sequences may be used in such methods to obtain a nucleic acid fragment encoding a substantial portion of an amino acid sequence of a polypeptide.

Availability of the instant nucleotide and deduced amino acid sequences facilitates immunological screening of cDNA expression libraries. Synthetic peptides representing substantial portions of the instant amino acid sequences may be synthesized. These peptides can be used to immunize animals to produce polyclonal or monoclonal antibodies with specificity for peptides or proteins comprising the amino acid sequences. These antibodies can be then be used to screen cDNA expression libraries to isolate full-length cDNA clones of interest (Lemer (1984) Adv. Immunol. 36:1-34; Maniatis).

In another embodiment, this invention concerns viruses and host cells comprising either the chimeric genes of the invention as described herein or an isolated polynucleotide of the invention as described herein. Examples of host cells which can be used to practice the invention include, but are not limited to, yeast, bacteria, and plants.

The nucleic acid fragments of the instant invention may be used to create transgenic plants in which the disclosed plant defensin are present at higher or lower levels than normal or in cell types or developmental stages in which they are not normally found. This would have the effect of altering the level of disease (e.g., fungal) resistance and stress tolerance in those cells.

Overexpression of the proteins of the instant invention may be accomplished by first constructing a chimeric gene in which the coding region is operably linked to a promoter capable of directing expression of a gene in the desired tissues at the desired stage of development. The chimeric gene may comprise promoter sequences and translation leader sequences derived from the same genes. 3' Non-coding sequences encoding transcription termination signals may also be provided. The instant chimeric gene may also comprise one or more introns in order to facilitate gene expression.

Plasmid vectors comprising the instant isolated polynucleotide (or chimeric gene) may be constructed. The choice of plasmid vector is dependent upon the method that will be used to transform host plants. The skilled artisan is well aware of the genetic elements that must be present on the plasmid vector in order to successfully transform, select and propagate host cells containing the chimeric gene. The skilled artisan will also recognize that different independent transformation events will result in different levels and patterns of expression (Jones et al. (1985) EMBO J. 4:2411-2418; De Almeida et al. (1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, Western analysis of protein expression, or phenotypic analysis.

For some applications it may be useful to direct the instant polypeptides to different cellular compartments, or to facilitate secretion from the cell. It is thus envisioned that the chimeric gene described above may be further supplemented by directing the coding sequence to encode the instant polypeptides with appropriate intracellular targeting sequences such as transit sequences (Keegstra (1989) Cell 56:247-253), signal sequences or sequences encoding endoplasmic reticulum localization (Chrispeels (1991) Ann. Rev. Plant Phys. Plant Mol. Biol. 42:21-53), or nuclear localization signals (Raikhel (1992) Plant Phys. 100: 1627-1632) with or without removing targeting sequences that are already present. While the references cited give examples of each of these, the list is not exhaustive and more targeting signals of use may be discovered in the future.

The instant polypeptides (or portions thereof) may be produced in heterologous host cells, particularly in the cells of microbial hosts, and can be used to prepare antibodies to the proteins by methods well known to those skilled in the art. The antibodies are useful for detecting the polypeptides of the instant invention *in situ* in cells or *in vitro* in cell extracts. Preferred heterologous host cells for production of the instant polypeptides are microbial hosts. Microbial expression systems and expression vectors containing regulatory sequences that direct high level expression of foreign proteins are well known to those skilled in the art. Any of these could be used to construct a chimeric gene for production of the instant polypeptides. This chimeric gene could then be introduced into appropriate microorganisms via transformation to provide high level expression of the encoded plant defensin. An example of a vector for high level expression of the instant polypeptides in a bacterial host is provided (Example 6).

All or a substantial portion of the polynucleotides of the instant invention may also be used as probes for genetically and physically mapping the genes that they are a part of, and used as markers for traits linked to those genes. Such information may be useful in plant breeding in order to develop lines with desired phenotypes. For example, the instant nucleic acid fragments may be used as restriction fragment length polymorphism (RFLP) markers. Southern blots (Maniatis) of restriction-digested plant genomic DNA may be probed with the nucleic acid fragments of the instant invention. The resulting banding patterns may then be subjected to genetic analyses using computer programs such as MapMaker (Lander et al. (1987) Genomics 1:174-181) in order to construct a genetic map. In addition, the nucleic acid fragments of the instant invention may be used to probe Southern blots containing restriction endonuclease-treated genomic DNAs of a set of individuals representing parent and progeny of a defined genetic cross. Segregation of the DNA polymorphisms is noted and used to calculate the position of the instant nucleic acid sequence in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4:37-41. Numerous publications describe genetic mapping of specific cDNA clones using the methodology outlined above or variations thereof For example, F2 intercross populations, backcross populations, randomly mated populations, near isogenic lines, and other sets of individuals may be used for mapping. Such methodologies are well known to those skilled in the art.

Nucleic acid probes derived from the instant nucleic acid sequences may also be used for physical mapping (i.e., placement of sequences on physical maps; *see* Hoheisel et al. In: Nonmammalian Genomic Analysis: A Practical Guide, Academic press 1996, pp. 319-346, and references cited therein).

In another embodiment, nucleic acid probes derived from the instant nucleic acid sequences may be used in direct fluorescence *in situ* hybridization (FISH) mapping (Trask (1991) Trends Genet. 7:149-154). Although current methods of FISH mapping favor use of large clones (several to several hundred KB; see Laan et al. (1995) Genome Res. 5:13-20), improvements in sensitivity may allow performance of FISH mapping using shorter probes.

A variety of nucleic acid amplification-based methods of genetic and physical mapping may be carried out using the instant nucleic acid sequences. Examples include allele-specific amplification (Kazazian (1989) J. Lab. Clin. Med. 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid fragment is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Loss of function mutant phenotypes may be identified for the instant cDNA clones either by targeted gene disruption protocols or by identifying specific mutants for these genes contained in a maize population carrying mutations in all possible genes (Ballinger and Benzer (1989) Proc. Natl. Acad Sci USA 86:9402-9406; Koes et al. (1995) Proc. Natl. Acad. Sci USA 92:8149-8153; Bensen et al. (1995) Plant Cell 7:75-84). The latter approach may be accomplished in two ways. First, short segments of the instant nucleic acid fragments may be used in polymerase chain reaction protocols in conjunction with a mutation tag sequence primer on DNAs prepared from a population of plants in which Mutator transposons or some other mutation-causing DNA element has been introduced (see Bensen, *supra*)*.* The amplification of a specific DNA fragment with these primers indicates the insertion of the mutation tag element in or near the plant gene encoding the instant polypeptide. Alternatively, the instant nucleic acid fragment may be used as a hybridization probe against PCR amplification products generated from the mutation population using the mutation tag sequence primer in conjunction with an arbitrary genomic site primer, such as that for a restriction enzyme site-anchored synthetic adaptor. With either method, a plant containing a mutation in the endogenous gene encoding the instant polypeptide can be identified and obtained. This mutant plant can then be used to determine or confirm the natural function of the instant polypeptides disclosed herein.

### EXAMPLES

The present invention is further defined in the following Examples, in which parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the scope thereof, can make various changes and modifications of the invention to adapt it to various usages and conditions. Thus, various modifications of the invention in addition to those shown and described herein will be apparent to those skilled in the art from the foregoing description. Such modifications are also intended to fall within the scope of the appended claims.

### EXAMPLE 1

### Composition of cDNA Libraries; Isolation and Sequencing of cDNA Clones

cDNA libraries representing mRNAs from various *Dimorphotheca sinuata, Picramnia pentandra, Parthenium argentatum* Grey, and *Nicotiana benthamiana* tissues were prepared. The characteristics of the libraries are described below.

**TABLE 2**

| cDNA Libraries from *Dimorphotheca sinuata, Picramnia pentandra, Parthenium argentatum* Grey, and *Nicotiana benthamiana* | | |
|---|---|---|
| Library | Tissue | Clone |
| dms2c | African Daisy *(Dimorphotheca sinuata)* Developing Seed | dms2c.pk001.d3 |
| epb1c | *Parthenium argentatum* Grey Stem Bark | epb1c.pk001.h15 |
| | | epb1c.pk002.h2 |
| | | epb1c.pk003.p14 |
| | | epb1c.pk004.p22 |
| | | epb1c.pk005.o6 |
| | | epb1c.pk006.k15 |
| epb3c | *Parthenium argentatum* Grey Stem Bark | epb3c.pk009.j22 |
| pps | Florida Bitterbush (*Picramnia pentandra)* Developing Seed | pps.pk0011.a9 |
| tdrlc | *Nicotiana benthamiana* Developing Root | tdr1c.pk002.g7 |

| | | |
|---|---|---|
| *This library was normalized essentially as described in U.S. Patent No. 5,482,845, incorporated herein by reference: | | |

cDNA libraries may be prepared by any one of many methods available. For example, the cDNAs may be introduced into plasmid vectors by first preparing the cDNA libraries in Uni-ZAP™ XR vectors according to the manufacturer's protocol (Stratagene Cloning Systems, La Jolla, CA). The Uni-ZAP™ XR libraries are converted into plasmid libraries according to the protocol provided by Stratagene. Upon conversion, cDNA inserts will be contained in the plasmid vector pBluescript. In addition, the cDNAs may be introduced directly into precut Bluescript II SK(+) vectors (Stratagene) using T4 DNA ligase (New England Biolabs), followed by transfection into DH10B cells according to the manufacturer's protocol (GIBCO BRL Products). Once the cDNA inserts are in plasmid vectors, plasmid DNAs are prepared from randomly picked bacterial colonies containing recombinant pBluescript plasmids, or the insert cDNA sequences are amplified via polymerase chain reaction using primers specific for vector sequences flanking the inserted cDNA sequences. Amplified insert DNAs or plasmid DNAs are sequenced in dye-primer sequencing reactions to generate partial cDNA sequences (expressed sequence tags or "ESTs"; see Adams et al., (1991) Science 252:1651-1656). The resulting ESTs are analyzed using a Perkin Elmer Model 377 fluorescent sequencer.

### EXAMPLE 2

### Identification of cDNA Clones

cDNA clones encoding plant defensin were identified by conducting BLAST (Basic Local Alignment Search Tool; Altschul et al. (1993) J. Mol. Biol. 215:403-410; see also www.ncbi.nlm.nih.gov/BLAST/) searches for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The cDNA sequences obtained in Example 1 were analyzed for similarity to all publicly available DNA sequences contained in the "nr" database using the BLASTN algorithm provided by the National Center for Biotechnology Information (NCBI). The DNA sequences were translated in all reading frames and compared for similarity to all publicly available protein sequences contained in the "nr" database using the BLASTX algorithm (Gish and States (1993) Nat. Genet. 3:266-272) provided by the NCBI. For convenience, the P-value (probability) of observing a match of a cDNA sequence to a sequence contained in the searched databases merely by chance as calculated by BLAST are reported herein as "pLog" values, which represent the negative of the logarithm of the reported P-value. Accordingly, the greater the pLog value, the greater the likelihood that the cDNA sequence and the BLAST "hit" represent homologous proteins.

### EXAMPLE 3

### Characterization of cDNA Clones Encoding Plant Defensin

The BLASTX search using the EST sequences from clones dms2c.pk001.d3, epblc.pk002.h2, pps.pk0011.a9 and vsln.pk0009.h6 revealed similarity of the proteins encoded by the cDNAs to defensin from *Dahlia merckii* (NCBI Gene Identifier No. 2147320). The BLAST results for each of these ESTs are shown in Table 3:

**TABLE 3**

| BLAST Results for Clones Encoding Polypeptides Homologous to Plant Defensin | |
|---|---|
| Clone | BLAST pLog Score 2147320 |
| dms2c.pk001.d3 | 30.4 |
| epb1c.pk002.h2 | 30.3 |
| pps.pk0011.a9 | 24.0 |

The BLASTX search using the EST sequence from clone tdr1c.pk002.g7 revealed similarity of the protein encoded by the cDNA to defensin from *Nicotiana tabacum* (NCBI Gene Identifier No. 676882) with a pLog value of 16.4.

The sequence of a portion of the cDNA insert from clone dms2c.pk001.d3 is shown in SEQ ID NO:1; the deduced amino acid sequence of this cDNA is shown in SEQ ID NO:2. The sequence of a portion of the cDNA insert from clone epb 1 c.pk002.h2 is shown in SEQ ID NO:9; the deduced amino acid sequence of this cDNA is shown in SEQ ID NO:10. The sequence of a portion of the cDNA insert from clone pps.pk0011.a9 is shown in SEQ ID NO:5; the deduced amino acid sequence of this cDNA is shown in SEQ ID NO:6. The sequence of a portion of the cDNA insert from clone tdrlc.pk002.g7 is shown in SEQ ID NO:25; the deduced amino acid sequence of this cDNA is shown in SEQ ID NO:26. BLAST scores and probabilities indicate that the instant nucleic acid fragments encode portions of plant defensin.

The BLASTX search using the EST sequences from clones listed in Table 4 revealed similarity of the polypeptides encoded by the cDNAs to a defensin from *Dahlia merckii* (NCBI GenBank Identifier (GI) No. 2147320: WO 99/02038-A1; Osborn et al. (1995) FEBS Lett 368:257-262). Shown in Table 4 are the BLAST results for individual ESTs ("EST"), the sequences of the entire cDNA inserts comprising the indicated cDNA clones ("FIS"), contigs assembled from two or more ESTs ("Contig"), contigs assembled from an FIS and one or more ESTs ("Contig*"), or sequences encoding at a minimum the entire mature protein derived from an EST, an FIS, a contig, or an FIS and PCR ("CGS"):

**TABLE 4**

| BLAST Results for Sequences Encoding Polypeptides Homologous to Plant Defensin | | |
|---|---|---|
| Clone | Status | BLAST pLog Score 2147320 |
| dms2c.pk001.d3(FIS) | CGS | 25.70 |
| pps.pk0011.a9(FIS) | CGS | 23.10 |
| epblc.pk002.h2(FIS) | CGS | 25.22 |
| epb1c.pk001.h15(EST) | CGS | 25.00 |
| epblc.pk003.p14(EST) | CGS | 30.22 |
| epb1c.pk004.p22(EST) | CGS | 30.22 |
| epb1c.pk005.o6(EST) | CGS | 30.22 |
| epb1c.pk006.k15(EST) | CGS | 30.22 |
| epb3c.pk009 j22(EST) | CGS | 30.22 |
| tdr1c.pk002.g7(FIS) | CGS | 11.70 |

Figure 1 presents an alignment of the amino acid sequences set forth in SEQ ID NOs:4, 8, 12, 14, 16, 18, 20, 22, 24, and 28 and the *Dahlia merckii* sequence (NCBI GenBank Identifier (GI) No. 2147320; SEQ ID NO:29). The data in Table 5 represents a calculation of the percent identity of the amino acid sequences set forth in SEQ ID NOs:4, 8, 12, 14, 16, 18, 20, 22, 24, and 28 and the *Dahlia merckii* sequence (NCBI GenBank Identifier (GI) No. 2147320; SEQ ID NO:29).

**TABLE 5**

| Percent Identity of Amino Acid Sequences Deduced From the Nucleotide Sequences of cDNA Clones Encoding Polypeptides Homologous to Plant Defensin | |
|---|---|
| SEQ ID NO. | Percent Identity to NCBI GenBank Identifier (GI) No. 2147320; SEQ ID NO:29 |
| 4 | 92.0 |
| 8 | 84.0 |
| 12 | 92.0 |
| 14 | 92.0 |
| 16 | 92.0 |
| 18 | 92.0 |
| 20 | 92.0 |
| 22 | 92.0 |
| 24 | 92.0 |
| 28 | 56.0 |

Sequence alignments and percent identity calculations were performed using the Megalign program of the LASERGENE bioinformatics computing suite (DNASTAR Inc., Madison, WI). Multiple alignment of the sequences was performed using the Clustal method of alignment (Higgins and Sharp (1989) CABIOS. 5:151-153) with the default parameters (GAP PENALTY=10, GAP LENGTH PENALTY=10). Default parameters for pairwise alignments using the Clustal method were KTUPLE 1, GAP PENALTY=3, WINDOW=5 and DIAGONALS SAVED=5. Sequence alignments, BLAST scores and probabilities indicate that the nucleic acid fragments comprising the instant cDNA clones encode entire plant defensins.

### EXAMPLE 4

### Expression of Chimeric Genes in Monocot Cells.

A chimeric gene comprising a cDNA encoding the instant polypeptide in sense orientation with respect to the maize 27 kD zein promoter that is located 5' to the cDNA fragment, and the 10 kD zein 3' end that is located 3' to the cDNA fragment, can be constructed. The cDNA fragment of this gene may be generated by polymerase chain reaction (PCR) of the cDNA clone using appropriate oligonucleotide primers. Cloning sites (NcoI or Smal) can be incorporated into the oligonucleotides to provide proper orientation of the DNA fragment when inserted into the digested vector pML103 as described below. Amplification is then performed in a standard PCR. The amplified DNA is then digested with restriction enzymes NcoI and SmaI and fractionated on an agarose gel. The appropriate band can be isolated from the gel and combined with a 4.9 kb NcoI-SmaI fragment of the plasmid pML 103. Plasmid pML 103 has been deposited under the terms of the Budapest Treaty at ATCC (American Type Culture Collection, 10801 University Blvd., Manassas, VA 20110-2209), and bears accession number ATCC 97366. The DNA segment from pML103 contains a 1.05 kb SalI-NcoI promoter fragment of the maize 27 kD zein gene and a 0.96 kb SmaI-SaII fragment from the 3' end of the maize 10 kD zein gene in the vector pGem9Zf(+) (Promega). Vector and insert DNA can be ligated at 15°C overnight, essentially as described (Maniatis). The ligated DNA may then be used to transform *E. coli* XL1-Blue (Epicurian Coli XL-1 Blues; Stratagene). Bacterial transformants can be screened by restriction enzyme digestion of plasmid DNA and limited nucleotide sequence analysis using the dideoxy chain termination method (Sequenase™ DNA Sequencing Kit; U.S. Biochemical). The resulting plasmid construct would comprise a chimeric gene encoding, in the 5' to 3' direction, the maize 27 kD zein promoter, a cDNA fragment encoding the instant polypeptide, and the 10 kD zein 3' region.

The chimeric gene described above can then be introduced into corn cells by the following procedure. Immature corn embryos can be dissected from developing caryopses derived from crosses of the inbred corn lines H99 and LH132. The embryos are isolated 10 to 11 days after pollination when they are 1.0 to 1.5 mm long. The embryos are then placed with the axis-side facing down and in contact with agarose-solidified N6 medium (Chu et al. (1975) Sci. Sin. Peking 18:659-668). The embryos are kept in the dark at 27°C. Friable embryogenic callus consisting of undifferentiated masses of cells with somatic proembryoids and embryoids borne on suspensor structures proliferates from the scutellum of these immature embryos. The embryogenic callus isolated from the primary explant can be cultured on N6 medium and sub-cultured on this medium every 2 to 3 weeks.

The plasmid, p35S/Ac (obtained from Dr. Peter Eckes, Hoechst Ag, Frankfurt, Germany) may be used in transformation experiments in order to provide for a selectable marker. This plasmid contains the *Pat* gene (see European Patent Publication 0 242 236) which encodes phosphinothricin acetyl transferase (PAT). The enzyme PAT confers resistance to herbicidal glutamine synthetase inhibitors such as phosphinothricin. The *pat* gene in p35S/Ac is under the control of the 35S promoter from Cauliflower Mosaic Virus (Odell et al. (1985) Nature 313:810-812) and the 3' region of the nopaline synthase gene from the T-DNA of the Ti plasmid of *Agrobacterium tumefaciens.*

The particle bombardment method (Klein et al. (1987) Nature 327:70-73) may be used to transfer genes to the callus culture cells. According to this method, gold particles (1 µm in diameter) are coated with DNA using the following technique. Ten µg of plasmid DNAs are added to 50 µL of a suspension of gold particles (60 mg per mL). Calcium chloride (50 µL of a 2.5 M solution) and spermidine free base (20 µL of a 1.0 M solution) are added to the particles. The suspension is vortexed during the addition of these solutions. After 10 minutes, the tubes are briefly centrifuged (5 sec at 15,000 rpm) and the supernatant removed. The particles are resuspended in 200 µL of absolute ethanol, centrifuged again and the supernatant removed. The ethanol rinse is performed again and the particles resuspended in a final volume of 30 µL of ethanol. An aliquot (5 µL) of the DNA-coated gold particles can be placed in the center of a Kapton™ flying disc (Bio-Rad Labs). The particles are then accelerated into the corn tissue with a Biolistic™ PDS-1000/He (Bio-Rad Instruments, Hercules CA), using a helium pressure of 1000 psi, a gap distance of 0.5 cm and a flying distance of 1.0 cm.

For bombardment, the embryogenic tissue is placed on filter paper over agarose-solidified N6 medium. The tissue is arranged as a thin lawn and covered a circular area of about 5 cm in diameter. The petri dish containing the tissue can be placed in the chamber of the PDS-1000/He approximately 8 cm from the stopping screen. The air in the chamber is then evacuated to a vacuum of 28 inches of Hg. The macrocarrier is accelerated with a helium shock wave using a rupture membrane that bursts when the He pressure in the shock tube reaches 1000 psi.

Seven days after bombardment the tissue can be transferred to N6 medium that contains gluphosinate (2 mg per liter) and lacks casein or proline. The tissue continues to grow slowly on this medium. After an additional 2 weeks the tissue can be transferred to fresh N6 medium containing gluphosinate. After 6 weeks, areas of about 1 cm in diameter of actively growing callus can be identified on some of the plates containing the glufosinate-supplemented medium. These calli may continue to grow when sub-cultured on the selective medium.

Plants can be regenerated from the transgenic callus by first transferring clusters of tissue to N6 medium supplemented with 0.2 mg per liter of 2,4-D. After two weeks the tissue can be transferred to regeneration medium (Fromm et al. (1990) BiolTechnology 8:833-839).

### EXAMPLE 5

### Expression of Chimeric Genes in Dicot Cells

A seed-specific construct composed of the promoter and transcription terminator from the gene encoding the β subunit of the seed storage protein phaseolin from the bean *Phaseolus vulgaris* (Doyle et al. (1986) J. Biol. Chem. 261:9228-9238) can be used for expression of the instant polypeptides in transformed soybean. The phaseolin construct includes about 500 nucleotides upstream (5') from the translation initiation codon and about 1650 nucleotides downstream (3') from the translation stop codon of phaseolin. Between the 5' and 3' regions are the unique restriction endonuclease sites Nco I (which includes the ATG translation initiation codon), Sma I, Kpn I and Xba I. The entire construct is flanked by Hind III sites.

The cDNA fragment of this gene may be generated by polymerase chain reaction (PCR) of the cDNA clone using appropriate oligonucleotide primers. Cloning sites can be incorporated into the oligonucleotides to provide proper orientation of the DNA fragment when inserted into the expression vector. Amplification is then performed as described above, and the isolated fragment is inserted into a pUC 18 vector carrying the seed construct.

Soybean embryos may then be transformed with the expression vector comprising sequences encoding the instant polypeptides. To induce somatic embryos, cotyledons, 3-5 mm in length dissected from surface sterilized, immature seeds of the soybean cultivar A2872, can be cultured in the light or dark at 26°C on an appropriate agar medium for 6-10 weeks. Somatic embryos which produce secondary embryos are then excised and placed into a suitable liquid medium. After repeated selection for clusters of somatic embryos which multiplied as early, globular staged embryos, the suspensions are maintained as described below.

Soybean embryogenic suspension cultures can be maintained in 35 mL liquid media on a rotary shaker, 150 rpm, at 26°C with florescent lights on a 16:8 hour day/night schedule. Cultures are subcultured every two weeks by inoculating approximately 35 mg of tissue into 35 mL of liquid medium.

Soybean embryogenic suspension cultures may then be transformed by the method of particle gun bombardment (Klein et al. (1987) Nature (London) 327:70-73, U.S. Patent No. 4,945,050). A DuPont Biolistic™ PDS 1000/HE instrument (helium retrofit) can be used for these transformations.

A selectable marker gene which can be used to facilitate soybean transformation is a chimeric gene composed of the 35S promoter from Cauliflower Mosaic Virus (Odell et al. (1985) Nature 313:810-812), the hygromycin phosphotransferase gene from plasmid pJR225 (from *E. coli;* Gritz et al.(1983) Gene 25:179-188) and the 3' region of the nopaline synthase gene from the T-DNA of the Ti plasmid of *Agrobacterium tumefaciens.* The seed construct comprising the phaseolin 5' region, the fragment encoding the instant polypeptide and the phaseolin 3' region can be isolated as a restriction fragment. This fragment can then be inserted into a unique restriction site of the vector carrying the marker gene.

To 50 µL of a 60 mg/mL 1 µm gold particle suspension is added (in order): 5 µL DNA (1 µg/µL), 20 µL spermidine (0.1 M), and 50 µL CaCl₂ (2.5 M). The particle preparation is then agitated for three minutes, spun in a microfuge for 10 seconds and the supernatant removed. The DNA-coated particles are then washed once in 400 µL 70% ethanol and resuspended in 40 µL of anhydrous ethanol. The DNA/particle suspension can be sonicated three times for one second each. Five µL of the DNA-coated gold particles are then loaded on each macro carrier disk.

Approximately 300-400 mg of a two-week-old suspension culture is placed in an empty 60x15 mm petri dish and the residual liquid removed from the tissue with a pipette. For each transformation experiment, approximately 5-10 plates of tissue are normally bombarded. Membrane rupture pressure is set at 1100 psi and the chamber is evacuated to a vacuum of 28 inches mercury. The tissue is placed approximately 3.5 inches away from the retaining screen and bombarded three times. Following bombardment, the tissue can be divided in half and placed back into liquid and cultured as described above.

Five to seven days post bombardment, the liquid media may be exchanged with fresh media, and eleven to twelve days post bombardment the liquid media may be exchanged with fresh media containing 50 mg/mL hygromycin. This selective media can be refreshed weekly. Seven to eight weeks post bombardment, green, transformed tissue may be observed growing from untransformed, necrotic embryogenic clusters. Isolated green tissue is removed and inoculated into individual flasks to generate new, clonally propagated, transformed embryogenic suspension cultures. Each new line may be treated as an independent transformation event. These suspensions can then be subcultured and maintained as clusters of immature embryos or regenerated into whole plants by maturation and germination of individual somatic embryos.

### EXAMPLE 6

### Expression of Chimeric Genes in Microbial Cells

The cDNAs encoding the instant polypeptides can be inserted into the T7 *E. coli* expression vector pBT430. This vector is a derivative of pET-3a (Rosenberg et al. (1987) Gene 56:125-135) which employs the bacteriophage T7 RNA polymerase/T7 promoter system. Plasmid pBT430 was constructed by first destroying the EcoR I and Hind III sites in pET-3a at their original positions. An oligonucleotide adaptor containing EcoR I and Hind III sites was inserted at the BamH I site of pET-3a. This created pET-3aM with additional unique cloning sites for insertion of genes into the expression vector. Then, the Nde I site at the position of translation initiation was converted to an Nco I site using oligonucleotide-directed mutagenesis. The DNA sequence of pET-3aM in this region, 5'-CATATGG, was converted to 5'-CCCATGG in pBT430.

Plasmid DNA containing a cDNA may be appropriately digested to release a nucleic acid fragment encoding the protein. This fragment may then be purified on a 1% NuSieve GTG™ low melting agarose gel (FMC). Buffer and agarose contain 10 µg/mL ethidium bromide for visualization of the DNA fragment. The fragment can then be purified from the agarose gel by digestion with GELase™ (Epicentre Technologies) according to the manufacturer's instructions, ethanol precipitated, dried and resuspended in 20 µL of water. Appropriate oligonucleotide adapters may be ligated to the fragment using T4 DNA ligase (New England Biolabs, Beverly, MA). The fragment containing the ligated adapters can be purified from the excess adapters using low melting agarose as described above. The vector pBT430 is digested, dephosphorylated with alkaline phosphatase (NEB) and deproteinized with phenol/chloroform as described above. The prepared vector pBT430 and fragment can then be ligated at 16°C for 15 hours followed by transformation into DH5 electrocompetent cells (GIBCO BRL). Transformants can be selected on agar plates containing LB media and 100 µg/mL ampicillin. Transformants containing the gene encoding the instant polypeptide are then screened for the correct orientation with respect to the T7 promoter by restriction enzyme analysis.

For high level expression, a plasmid clone with the cDNA insert in the correct orientation relative to the T7 promoter can be transformed into *E. coli* strain BL21 (DE3) (Studier et al. (1986) J. Mol. Biol. 189: 113-130). Cultures are grown in LB medium containing ampicillin (100 mg/L) at 25°C. At an optical density at 600 nm of approximately 1, IPTG (isopropylthio-β-galactoside, the inducer) can be added to a final concentration of 0.4 mM and incubation can be continued for 3 h at 25°. Cells are then harvested by centrifugation and re-suspended in 50 µL of 50 mM Tris-HCl at pH 8.0 containing 0.1 mM DTT and 0.2 mM phenyl methylsulfonyl fluoride. A small amount of 1 mm glass beads can be added and the mixture sonicated 3 times for about 5 seconds each time with a microprobe sonicator. The mixture is centrifuged and the protein concentration of the supernatant determined. One µg of protein from the soluble fraction of the culture can be separated by SDS-polyacrylamide gel electrophoresis. Gels can be observed for protein bands migrating at the expected molecular weight.

### EXAMPLE 7

### Assaying Plant Defensin Activity

The polypeptides described herein may be produced using any number of methods known to those skilled in the art. Such methods include, but are not limited to, expression in bacteria as described in Example 6, or expression in eukaryotic cell culture, *in planta,* and using viral expression systems in suitably infected organisms or cell lines. The instant polypeptides may be expressed either as mature forms of the proteins as observed *in vivo* or as fusion proteins by covalent attachment to a variety of enzymes, proteins or affinity tags. Common fusion protein partners include glutathione S-transferase ("GST"), thioredoxin ("Trx"), maltose binding protein, and C- and/or N-terminal hexahistidine polypeptide ("(His)₆"). The fusion proteins may be engineered with a protease recognition site at the fusion point so that fusion partners can be separated by protease digestion to yield intact mature enzyme. Examples of such proteases include thrombin, enterokinase and factor Xa. However, any protease can be used which specifically cleaves the peptide connecting the fusion protein and the enzyme.

Purification of the instant polypeptides, if desired, may utilize any number of separation technologies familiar to those skilled in the art of protein purification. Examples of such methods include, but are not limited to, homogenization, filtration, centrifugation, heat denaturation, ammonium sulfate precipitation, desalting, pH precipitation, ion exchange chromatography, hydrophobic interaction chromatography and affinity chromatography, wherein the affinity ligand represents a substrate, substrate analog or inhibitor. When the instant polypeptides are expressed as fusion proteins, the purification protocol may include the use of an affinity resin which is specific for the fusion protein tag attached to the expressed enzyme or an affinity resin containing ligands which are specific for the enzyme. For example, the instant polypeptides may be expressed as a fusion protein coupled to the C-terminus of thioredoxin. In addition, a (His)₆ peptide may be engineered into the N-terminus of the fused thioredoxin moiety to afford additional opportunities for affinity purification. Other suitable affinity resins could be synthesized by linking the appropriate ligands to any suitable resin such as Sepharose-4B. In an alternate embodiment, a thioredoxin fusion protein may be eluted using dithiothreitol; however, elution may be accomplished using other reagents which interact to displace the thioredoxin from the resin. These reagents include β-mercaptoethanol or other reduced thiol. The eluted fusion protein may be subjected to further purification by traditional means as stated above, if desired. Proteolytic cleavage of the thioredoxin fusion protein and the enzyme may be accomplished after the fusion protein is purified or while the protein is still bound to the ThioBond" affinity resin or other resin.

Crude, partially purified or purified enzyme, either alone or as a fusion protein, may be utilized in assays to verify over- or underexpression of functional plant defensins in transgenic plants and transformed bacterial cells. Assays may be conducted under well known experimental conditions which permit optimal enzymatic activity. For example, assays for plant defensin are presented by Thevissen, K. et al. (1996) J. Biol. Chem. 271(25):15018-15025.

## Claims

1. An isolated polynucleotide encoding a defensin polypeptide having antimicrobial activity and comprising a nucleotide sequence selected from:
(a) a nucleotide sequence encoding a polypeptide of at least 49 amino acids that has at least 95% identity based on the Clustal method of alignment when compared to a polypeptide of SEQ ID NO: 8; and
(b) a nucleotide sequence encoding a polypeptide of at least 49 amino acids that has at least 90% identity based on the Clustal method of alignment when compared to a polypeptide of SEQ ID NO: 6.

2. The isolated polynucleotide of claim 1, part (b), wherein said percentage identity is at least 95%.

3. The isolated polynucleotide of claim 1, wherein the nucleotide sequence comprises a nucleic acid sequence of SEQ ID NOs: 5 or 7 that codes for the polypeptide of SEQ ID NOs: 6 or 8.

4. The isolated polynucleotide of claim 1 wherein the nucleotide sequences are DNA.

5. The isolated polynucleotide of claim 1 wherein the nucleotide sequences are RNA.

6. A chimeric gene comprising the isolated polynucleotide of claim 1 operably linked to at least one suitable regulatory sequence.

7. A host cell comprising the chimeric gene of claim 6.

8. A host cell comprising the isolated polynucleotide of claim 1.

9. The host cell of claim 7 wherein the host cell is selected from yeast, bacteria, and plant.

10. A virus comprising the isolated polynucleotide of claim 1.

11. A defensin polypeptide having antimicrobial activity and selected from:
(a) a polypeptide of at least 49 amino acids that has at least 90% identity based on the Clustal method of alignment when compared to a polypeptide of SEQ ID NO: 6; and
(b) a polypeptide of at least 50 amino acids that has at least 95% identity based on the Clustal method of alignment when compared to a polypeptide of SEQ ID NO: 8.

12. The polypeptide of claim 11, part (a) wherein said percentage identity is at least 95%.

13. A composition comprising the isolated polynucleotide of claim 1.

14. A composition comprising the isolated polypeptide of claim 11.

15. A method for positive selection of a transformed cell comprising:
(a) transforming a host cell with the chimeric gene of Claim 6; and
(b) growing the transformed host cell under conditions which allow expression of the polynucleotide in an amount sufficient to complement a null mutant to provide a positive selection means.

16. The method of claim 15 wherein the host cell is a plant.

17. The method of claim 16 wherein the plant cell is a monocot.

18. The method of claim 16 wherein the plant cell is a dicot.

19. A plant transformed with a polynucleotide of claim 1, 2 or 3 such that the polypeptide having antimicrobial activity is expressed to a level that exceeds the level of production in a non-transformed plant.

20. A plant of claim 19 which has increased levels of disease resistance and stress tolerance compared to said non-transformed plant.

21. A method of producing a plant of claim 19 or 20 comprising stably transferring a polynucleotide of claim 1 in the genome of a plant host cell and establishing a transgenic plant therefrom.

## Patentansprüche

1. Isoliertes Polynukleotid, das ein Defensin-Polypeptid mit antimikrobieller Aktivität codiert und eine Nukleotidsequenz umfasst, die ausgewählt ist aus:
(a) einer Nukleotidsequenz, die ein Polypeptid aus wenigstens 49 Aminosäuren codiert, welches wenigstens 95% Identität, basierend auf der Clustal-Alignment-Methode, hat, wenn man es mit einem Polypeptid von SEQ ID NO:8 vergleicht, und
(b) einer Nukleotidsequenz, die ein Polypeptid aus wenigstens 49 Aminosäuren codiert, welches wenigstens 90% Identität, basierend auf der Clustal-Alignment-Methode, hat, wenn man es mit einem Polypeptid von SEQ ID NO:6 vergleicht.

2. Isoliertes Polynukleotid gemäß Anspruch 1, Teil (b), wobei die prozentuale Identität wenigstens 95% ist.

3. Isoliertes Polynukleotid gemäß Anspruch 1, wobei die Nukleotidsequenz eine Nukleinsäuresequenz von SEQ ID NO:5 oder 7 umfasst, die für das Polypeptid von SEQ ID NO:6 oder 8 codiert.

4. Isoliertes Polynukleotid gemäß Anspruch 1, wobei die Nukleotidsequenzen DNA sind.

5. Isoliertes Polynukleotid gemäß Anspruch 1, wobei die Nukleotidsequenzen RNA sind.

6. Chimäres Gen, umfassend das isolierte Polynukleotid gemäß Anspruch 1 funktionell verknüpft mit wenigstens einer geeigneten regulatorischen Sequenz.

7. Wirtszelle, die das chimäre Gen gemäß Anspruch 6 umfasst.

8. Wirtszelle, die das isolierte Polynukleotid gemäß Anspruch 1 umfasst.

9. Wirtszelle gemäß Anspruch 7, wobei die Wirtszelle aus Hefe, Bakterien und Pflanze ausgewählt ist.

10. Virus, das das isolierte Polynukleotid gemäß Anspruch 1 umfasst.

11. Defensin-Polypeptid mit antimikrobieller Aktivität und ausgewählt aus:
(a) einem Polypeptid aus wenigstens 49 Aminosäuren, das wenigstens 90% Identität, basierend auf der Clustal-Alignment-Methode, hat, wenn es mit einem Polypeptid von SEQ ID NO:6 verglichen wird, und
(b) einem Polypeptid aus wenigstens 50 Aminosäuren, das wenigstens 95% Identität, basierend auf der Clustal-Alignment-Methode, hat, wenn es mit einem Polypeptid von SEQ ID NO:8 verglichen wird.

12. Polypeptid gemäß Anspruch 11, Teil (a), wobei die prozentuale Identität wenigstens 95% ist.

13. Zusammensetzung, die das isolierte Polynukleotid gemäß Anspruch 1 umfasst.

14. Zusammensetzung, die das isolierte Polypeptid gemäß Anspruch 11 umfasst.

15. Verfahren zur positiven Selektion einer transformierten Zelle, umfassend:
(a) Transformieren einer Wirtszelle mit dem chimären Gen gemäß Anspruch 6 und
(b) Wachsenlassen der transformierten Wirtszelle unter Bedingungen, welche eine Expression des Polynukleotids in einer Menge erlauben, die ausreichend ist, um eine Nullmutante zu komplementieren, um ein positives Selektionsmittel bereitzustellen.

16. Verfahren gemäß Anspruch 15, wobei die Wirtszelle eine Pflanze ist.

17. Verfahren gemäß Anspruch 16, wobei die Pflanzenzelle eine Monokotyle ist.

18. Verfahren gemäß Anspruch 16, wobei die Pflanzenzelle eine Dikotyle ist.

19. Pflanze, die mit einem Polynukleotid gemäß Anspruch 1, 2 oder 3 transformiert ist, so dass das Polypeptid mit antimikrobieller Aktivität zu einem Level exprimiert wird, der den Level der Produktion in einer nichttransformierten Pflanze übersteigt.

20. Pflanze gemäß Anspruch 19, welche erhöhte Level an Krankheitsresistenz und Stresstoleranz, verglichen mit der nichttransformierten Pflanze, hat.

21. Verfahren zur Produktion einer Pflanze gemäß Anspruch 19 oder 20, umfassend stabiles Transferieren eines Polynukleotids gemäß Anspruch 1 in das Genom einer Pflanzenwirtszelle und Entwickeln daraus einer transgenen Pflanze.

## Revendications

1. Polynucléotide isolé codant pour un polypeptide de défensine ayant une activité antimicrobienne et comprenant une séquence nucléotidique choisie parmi:
(a) une séquence nucléotidique codant pour un polypeptide d'au moins 49 acides aminés qui a au moins 95 % d'identité en se basant sur la méthode d'alignement de Clustal lorsqu'il est comparé à un polypeptide de SEQ ID NO : 8 ; et
(b) une séquence nucléotidique codant pour un polypeptide d'au moins 49 acides aminés qui a au moins 90 % d'identité en se basant sur la méthode d'alignement de Clustal lorsqu'il est comparé à un polypeptide de SEQ ID NO : 6.

2. Polynucléotide isolé de la revendication 1, partie (b), dans lequel ledit pourcentage d'identité est au moins de 95 %.

3. Polynucléotide isolé de la revendication 1, dans lequel la séquence nucléotidique comprend une séquence d'acide nucléique de SEQ ID NO : 5 ou 7 qui code pour le polypeptide de SEQ ID NO : 6 ou 8.

4. Polynucléotide isolé de la revendication 1, dans lequel les séquences nucléotidiques sont de l'ADN.

5. Polynucléotide isolé de la revendication 1, dans lequel les séquences nucléotidiques sont de l'ARN.

6. Gène chimère comprenant le polynucléotide isolé de la revendication 1 lié de manière fonctionnelle à au moins une séquence régulatrice appropriée.

7. Cellule hôte comprenant le gène chimère de la revendication 6.

8. Cellule hôte comprenant le polynucléotide isolé de la revendication 1.

9. Cellule hôte de la revendication 7 dans laquelle la cellule hôte est choisie parmi une levure, une bactérie et une plante.

10. Virus comprenant le polynucléotide isolé de la revendication 1.

11. Polypeptide de défensine ayant une activité antimicrobienne et choisi parmi :
(a) un polypeptide d'au moins 49 acides aminés qui a au moins 90 % d'identité en se basant sur la méthode d'alignement de Clustal lorsqu'il est comparé à un polypeptide de SEQ ID NO : 6 ; et
(b) un polypeptide d'au moins 50 acides aminés qui a au moins 95 % d'identité en se basant sur la méthode d'alignement de Clustal lorsqu'il est comparé à un polypeptide de SEQ ID NO : 8.

12. Polypeptide de la revendication 11, partie (a) dans lequel ledit pourcentage d'identité est au moins de 95 %.

13. Composition comprenant le polynucléotide isolé de la revendication 1.

14. Composition comprenant le polypeptide isolé de la revendication 11.

15. Méthode de sélection positive d'une cellule transformée comprenant :
(a) la transformation d'une cellule hôte avec le gène chimère de la revendication 6 ; et
(b) la culture de la cellule hôte transformée dans des conditions qui permettent l'expression du polynucléotide en quantité suffisante pour complémenter un mutant nul afin de fournir un moyen de sélection positif.

16. Méthode de la revendication 15 dans laquelle la cellule hôte est une plante.

17. Méthode de la revendication 16 dans laquelle la cellule végétale est une monocotylédone.

18. Méthode de la revendication 16 dans laquelle la cellule végétale est une dicotylédone.

19. Plante transformée avec un polynucléotide de la revendication 1, 2 ou 3 de telle façon que le polypeptide ayant une activité antimicrobienne soit exprimé à un niveau qui excède le niveau de production dans une plante non transformée.

20. Plante de la revendication 19 qui a des niveaux accrus de résistance à une maladie et de tolérance au stress comparée avec ladite plante non transformée.

21. Méthode de production d'une plante de la revendication 19 ou 20 comprenant le transfert stable d'un polynucléotide de la revendication 1 dans le génome d'une cellule végétale hôte et générant une plante transgénique à partir de celle-ci.


### SEQUENCE LISTING

<110> E. I. du Pont de Nemours and Company
<120> Plant Defensins
<130> BB1353
<140>
   <141>
<150> 60/133,039
   <151> 1999-05-07
<160> 29
<170> Microsoft Office 97
<210> 1
   <211> 503
   <212> DNA
   <213> Dimorphotheca sinuata
<220>
   <221> unsure
   <222> (321)
<220>
   <221> unsure
   <222> (349)
<220>
   <221> unsure
   <222> (416)
<220>
   <221> unsure
   <222> (458)
<220>
   <221> unsure
   <222> (474)
<220>
   <221> unsure
   <222> (479)
<220>
   <221> unsure
   <222> (482)
<220>
   <221> unsure
   <222> (502)
<400> 1
   ccttatcgtg attcaaaatg atgaaaagat cggttgctct ttccacatgc actttaattc  60
   ttttcgtgct cactatctca gaaatcgcga ctgtgagaag tgcactatgt gagaaagcta 120
   gcaagacatg gtcaggcaac tgtggcaaca cgggacactg tgacgaccag tgtaagtcgt 180
   gggagactgc agcccatggt gcgtgtcatg tgcgtggtgg gaaacacatg tgcttctgct 240
   acttcaattg taaagaagcc gaaaagcttg cccaagacaa gctcaacgct gaaaaattcg 300
   gccgtgatga cgttaaagta ntgtcggata tcaagaatcc atgaaaggnt tagtttcctt 360
   agacacgaaa aatctcagat taaaataaat aatagaataa ataaattatt aaatanaaat 420
   tgcttgttga ttgtcaaaaa aaaaaaaaaa aaaactcnag gggggggccg gtanccaant 480
   cnccctaaaa ggagtcgtat tna                                         503
<210> 2
   <211> 49
   <212> PRT
   <213> Dimorphotheca sinuata
<400> 2
   Leu Cys Glu Lys Ala Ser Lys Thr Trp Ser Gly Asn Cys Gly Asn Thr
   1               5                   10                  15
   Gly His Cys Asp Asp Gln Cys Lys Ser Trp Glu Thr Ala Ala His Gly
               20                  25                  30
   Ala Cys His Val Arg Gly Gly Lys His Met Cys Phe Cys Tyr Phe Asn
           35                  40                  45
   Cys
<210> 3
   <211> 460
   <212> DNA
   <213> Dimorphotheca sinuata
<400> 3
   gcacgagcct tatcgtgatt caaaatgatg aaaagatcgg ttgctctttc cacatgcact  60
   ttaattcttt tcgtgctcac tatctcagaa atcgcgactg tgagaagtgc actatgtgag 120
   aaagctagca agacatggtc aggcaactgt ggcaacacgg gacactgtga cgaccagtgt 180
   aagtcgtggg agactgcagc ccatggtgcg tgtcatgtgc gtggtgggaa acacatgtgc 240
   ttctgctact tcaattgtaa agaagccgaa aagcttgccc aagacaagct caacgctgaa 300
   aaattcggcc gtgatgacgt taaagtagtg tcggatatca agaatccatg aaaggattag 360
   tttccttaga cacgaaaaat ctcagattaa aataaataat agaataaata aattattaaa 420
   tagaaattgc ttgttgattg tcaaaaaaaa aaaaaaaaaa                       460
<210> 4
   <211> 108
   <212> PRT
   <213> Dimorphotheca sinuata
<400> 4
   Met Met Lys Arg Ser Val Ala Leu Ser Thr Cys Thr Leu Ile Leu Phe
   1               5                   10                  15
   Val Leu Thr Ile Ser Glu Ile Ala Thr Val Arg Ser Ala Leu Cys Glu
               20                  25                  30
   Lys Ala Ser Lys Thr Trp Ser Gly Asn Cys Gly Asn Thr Gly His Cys
           35                  40                  45
   Asp Asp Gln Cys Lys Ser Trp Glu Thr Ala Ala His Gly Ala Cys His
       50                  55                  60
   Val Arg Gly Gly Lys His Met Cys Phe Cys Tyr Phe Asn Cys Lys Glu
   65                  70                  75                  80
   Ala Glu Lys Leu Ala Gln Asp Lys Leu Asn Ala Glu Lys Phe Gly Arg
                   85                  90                  95
   Asp Asp Val Lys Val Val Ser Asp Ile Lys Asn Pro
               100                 105
<210> 5
   <211> 501
   <212> DNA
   <213> Picramnia pentandra
<220>
   <221> unsure
   <222> (10)
<220>
   <221> unsure
   <222> (14)
<220>
   <221> unsure
   <222> (215)
<220>
   <221> unsure
   <222> (240)
<220>
   <221> unsure
   <222> (283)
<220>
   <221> unsure
   <222> (311)
<220>
   <221> unsure
   <222> (356)
<220>
   <221> unsure
   <222> (407)
<220>
   <221> unsure
   <222> (444)
<220>
   <221> unsure
   <222> (451)
<220>
   <221> unsure
   <222> (455)
<220>
   <221> unsure
   <222> (484)
<220>
   <221> unsure
   <222> (501)
<400> 5
   cagcaaatan caancttaaa ttaaaagcag aaaaatggcc aaaccagcaa ccattcttgc  60
   catcctgttt gcctcttttg tcattcttgc ttcgtttgag agttccatgg gagcaagatc 120
   tacagaagag aaacccgagg ccgtaccaga ggctgagcag accgtagggg atcaagtcaa 180
   tgcagaagct gacacagtta tagacccgga ccaangacta tgtgaaagag caagcttaan 240
   atggtcaggc aattgtggca acactgctca ctgtgacaac cantgtaagt catgggagca 300
   cgcacaacac ngagcatgtc acgtacgagg tggaaaacat aagtgcttct gctacntcaa 360
   ttgctgatct caaagaaagc accttcccaa tgggtgcaac aagtaanaat gttaaaataa 420
   aataatttta gacatcaata cacngtctaa naatncttaa caatccttat ggttaagata 480
   cttnagtgct atcagtgaaa n                                          501
<210> 6
   <211> 49
   <212> PRT
   <213> Picramnia pentandra
<220>
   <221> UNSURE
   <222> (8)
<220>
   <221> UNSURE
   <222> (22)
<220>
   <221> UNSURE
   <222> (32)
<220>
   <221> UNSURE
   <222> (47)
<400> 6
   Leu Cys Glu Arg Ala Ser Leu Xaa Trp Ser Gly Asn Cys Gly Asn Thr
   1               5                   10                  15
   Ala His Cys Asp Asn Xaa Cys Lys Ser Trp Glu His Ala Gln His Xaa
               20                  25                  30
   Ala Cys His Val Arg Gly Gly Lys His Lys Cys Phe Cys Tyr Xaa Asn
           35                  40                  45
   Cys
<210> 7
   <211> 658
   <212> DNA
   <213> Picramnia pentandra
<400> 7
   gcacgagcag caaatagcaa acttaaatta aaagcagaaa aatggccaaa ccagcaacca  60
   ttcttgccat cctgtttgcc tcttttgtca ttcttgcttc gtttgagagt tccatgggag 120
   caagatctac agaagagaaa cccgaggccg taccagaggc tgagcagacc gtaggggatc 180
   aagtcaatgc agaagctgac acagttatag acccggacca aagactatgt gaaagagcaa 240
   gcttaacatg gtcaggcaat tgtggcaaca ctgctcactg tgacaaccag tgtaggtcat 300
   gggagcacgc acaacacgga gcatgtcacg tacgaggtgg aaaacatatg tgcttctgct 360
   acttcaattg ctgatctcaa agaaagcatc gttcgccaat ggttgcaaca agtaataatg 420
   ttaaaaataa aaataagtat agaccatcaa tacacggtct aagaattctt aacaatcctt 480
   attgttaaga ttacgttaag tgctatcagt gaaactagga ttgtcaccca actatgttcc 540
   agcaagctgg ctctgttgtt cttattttcc agtggttaat aaaaagattg tccaactctt 600
   ttacactaaa aaaaaaaaaa aaaaaaaaaa aaaaaaaaaa aaaaaaaaaa aaaaaaaa   658
<210>
   <211> 110
   <212> PRT
   <213> Picramnia pentandra
<400> 8
<210> 9
   <211> 463
   <212> DNA
   <213> Parthenium argentatum Grey
<220>
   <221> unsure
   <222> (413)
<400> 9
   caagtgttct tgagcttcag tgcattgatc caaaatgacc aaaacttcag ttgctttctt   60
   tgcatttctt ctgctcctcg ttcttgctat ctcagaaatc ggatcggtga agggagaact  120
   atgtgagaag gcaagcaaga catggtctgg aaattgtggt aacacaagac attgtgacga  180
   ccaatgcaag tcttgggagg gtgcagccca tggagcttgt catgtgcgcg gtgggaaaca  240
   catgtgcttc tgctacttcc agtgccccaa agccgagaag atggcccagg ataaactccg  300
   agctgaagag cttgccaagg agaagattga agctgaaaaa gagccaacca aaccttgagt  360
   agcaaatgtt atgcttatga ataagagaaa atgctttcta cttacatgtt cancaatttc  420
   taaggggtaa tgtttcctgc aattgggatc aattgttatg att                     463
<210> 10
   <211> 50
   <212> PRT
   <213> Parthenium argentatum Grey
<400> 10
   Glu Leu Cys Glu Lys Ala Ser Lys Thr Trp Ser Gly Asn Cys Gly Asn
   1 5 10 15
   Thr Arg His Cys Asp Asp Gln Cys Lys Ser Trp Glu Gly Ala Ala His
   20 25 30
   Gly Ala Cys His Val Arg Gly Gly Lys His Met Cys Phe Cys Tyr Phe
   35 40 45
   Gln Cys
   50
<210> 11
   <211> 603
   <212> DNA
   <213> Parthenium argentatum Grey
<400> 11
   gcacgagcaa gtgttcttga gcttcagtgc attgatccaa aatgaccaaa acttcagttg  60
   ctttctttgc atttcttctg ctcctcgttc ttgctatctc agaaatcgga tcggtgaagg 120
   gagaactatg tgagaaggca agcaagacat ggtctggaaa ttgtggtaac acaagacatt 180
   gtgacgacca atgcaagtct tgggagggtg cagcccatgg agcttgtcat gtgcgcggtg 240
   ggaaacacat gtgcttctgc tacttccagt gccccaaagc cgagaagatg gcccaggata 300
   aactccgagc tgaagagctt gccaaggaga agattgaagc tgaaaaagag ccagccaaac 360
   cttgagtagc aaatgttatg cttatgaata agagaaaatg ctttctactt acatgttcag 420
   cattttctat ggtgtaatgt ttcttgcatt tggaatcaat tgttatgatt ccttgtaaaa 480
   tgttctaatg atgtaataag tttcctgcat aaattatgtt gttttaaagc ctgtggaata 540
   aaattttatg tgcatgcaaa aaaaaaaaaa aaaaaaaaaa aaaaaaaaaa aaaaaaaaaa 600
   aaa                                                               603
<210> 12
   <211> 107
   <212> PRT
   <213> Parthenium argentatum Grey
<400> 12
   Met Thr Lys Thr Ser Val Ala Phe Phe Ala Phe Leu Leu Leu Leu Val
   1               5                   10                  15
   Leu Ala Ile Ser Glu Ile Gly Ser Val Lys Gly Glu Leu Cys Glu Lys
               20                  25                  30
   Ala Ser Lys Thr Trp Ser Gly Asn Cys Gly Asn Thr Arg His Cys Asp
           35                  40                  45
   Asp Gln Cys Lys Ser Trp Glu Gly Ala Ala His Gly Ala Cys His Val
       50                  55                  60
   Arg Gly Gly Lys His Met Cys Phe Cys Tyr Phe Gln Cys Pro Lys Ala
   65                  70                  75                  80
   Glu Lys Met Ala Gln Asp Lys Leu Arg Ala Glu Glu Leu Ala Lys Glu
                   85                  90                  95
   Lys Ile Glu Ala Glu Lys Glu Pro Ala Lys Pro
               100                 105
<210> 13
   <211> 457
   <212> DNA
   <213> Parthenium argentatum Grey
<220>
   <221> unsure
   <222> (424)
<220>
   <221> unsure
   <222> (436)
<400> 13
   gtgttcttga gcttcagtgc attgatccaa aatgaccaaa acttcagttg ctttctttgc  60
   atttcttctg ctcctcgttc ttgctatctc agaaatcgga tcggtgaagg gagaactatg 120
   tgagaaggca agcaagacat ggtctggaaa ttgtggtaac acaagacatt gtgacgacca 180
   atgcaagtct tgggagggtg cagcccatgg agcttgtcat gtgcgcggtg ggaaacacat 240
   gtgcttctgc tacttccagt gccccaaagc cgagaagatg gcccaggata aactccgagc 300
   tgaagagctt gccaaggaga agattgaagc tgaaaaagag ccaaccaaac cttgagtagc 360
   aaatgttatg cttatgaata aaagaaaatg ctttctactt acatgttcaa caatttccta 420
   tggngtaatg tttccntgca tttgggaatc aaattgg                          457
<210> 14
   <211> 107
   <212> PRT
   <213> Parthenium argentatum Grey
<400> 14
   Met Thr Lys Thr Ser Val Ala Phe Phe Ala Phe Leu Leu Leu Leu Val
   1               5                   10                  15
   Leu Ala Ile Ser Glu Ile Gly Ser Val Lys Gly Glu Leu Cys Glu Lys
               20                  25                  30
   Ala Ser Lys Thr Trp Ser Gly Asn Cys Gly Asn Thr Arg His Cys Asp
           35                  40                  45
   Asp Gln Cys Lys Ser Trp Glu Gly Ala Ala His Gly Ala Cys His Val
       50                  55                  60
   Arg Gly Gly Lys His Met Cys Phe Cys Tyr Phe Gln Cys Pro Lys Ala
   65                  70                  75                  80
   Glu Lys Met Ala Gln Asp Lys Leu Arg Ala Glu Glu Leu Ala Lys Glu
                   85                  90                  95
   Lys Ile Glu Ala Glu Lys Glu Pro Thr Lys Pro
               100                 105
<210> 15
   <211> 439
   <212> DNA
   <213> Parthenium argentatum Grey
<220>
   <221> unsure
   <222> (276)
<220>
   <221> unsure
   <222> (288)
<220>
   <221> unsure
   <222> (349)
<220>
   <221> unsure
   <222> (438)
<400> 15
   aagtgttctt gagcttcagt gcattgatcc aaaatgacca aaacttcagt tgctttcttt  60
   gcatttcttc tgctcctcgt tcttgctatc tcagaaatcg gatcggtgaa gggagaacta 120
   tgtgagaagg caagcaagac atggtctgga aattgtggta acacaagaca ttgtgacgac 180
   caatgcaagt cttgggaggg tgcagcccat ggagcttgtc atgtgcgcgg tgggaaacac 240
   atgtgcttct gctacttcca gtgccccaaa gccganaaga tggcccangg ataaactccg 300
   agctgaagaa gcttgccaag gagaagattg aagctgaaaa agagccagnc aaaccttgag 360
   taagcaaatg tttatgctta tgaaataaag aagaaaatgc ttttctactt tacatgttca 420
   gcaatttcct aagggggna                                              439
<210> 16
   <211> 86
   <212> PRT
   <213> Parthenium argentatum Grey
<220>
   <221> UNSURE
   <222> (81)
<220>
   <221> UNSURE
   <222> (85)
<400> 16
   Met Thr Lys Thr Ser Val Ala Phe Phe Ala Phe Leu Leu Leu Leu Val
   1               5                   10                  15
   Leu Ala Ile Ser Glu Ile Gly Ser Val Lys Gly Glu Leu Cys Glu Lys
               20                  25                  30
   Ala Ser Lys Thr Trp Ser Gly Asn Cys Gly Asn Thr Arg His Cys Asp
           35                  40                  45
   Asp Gln Cys Lys Ser Trp Glu Gly Ala Ala His Gly Ala Cys His Val
       50                  55                  60
   Arg Gly Gly Lys His Met Cys Phe Cys Tyr Phe Gln Cys Pro Lys Ala
   65                  70                  75                  80
   Xaa Lys Met Ala Xaa Gly
                   85
<210> 17
   <211> 460
   <212> DNA
   <213> Parthenium argentatum Grey
<220>
   <221> unsure
   <222> (342)
<400> 17
   gtttttgagc ttcagtgcat tgatccaaaa tggccaaaac ttcagttgct ttctttgcat  60
   ttcttctgct cctcgttctt gctatctcag aaatcggatc ggtgaaggga gaactatgtg 120
   agaaggcaag caagacatgg tctggaaatt gtggtaacac aagacactgt gacgaccaat 180
   gcaagtcttg ggagggtgca gcccatggag cttgtcatgt gcgcggtggg aaacacatgt 240
   gcttctgcta cttccagtgc cccaaagccg agaagatggc ccaggataaa ctccgagctg 300
   aagagcttgc caaggagaag attgaagctg aaaaagagcc anccaaacct tgagtagcaa 360
   atgttatgct tatgaataaa aagaaaatgc tttctactta catctttagc aatttctaag 420
   gggtaatgtt tcctgcattt gggatcaaat tgttatgatc                       460
<210> 18
   <211> 107
   <212> PRT
   <213> Parthenium-argentatum Grey
<220>
   <221> UNSURE
   <222> (105)
<400> 18
   Met Ala Lys Thr Ser Val Ala Phe Phe Ala Phe Leu Leu Leu Leu Val
   1               5                   10                  15
   Leu Ala Ile Ser Glu Ile Gly Ser Val Lys Gly Glu Leu Cys Glu Lys
               20                  25                  30
   Ala Ser Lys Thr Trp Ser Gly Asn Cys Gly Asn Thr Arg His Cys Asp
           35                  40                  45
   Asp Gln Cys Lys Ser Trp Glu Gly Ala Ala His Gly Ala Cys His Val
       50                  55                  60
   Arg Gly Gly Lys His Met Cys Phe Cys Tyr Phe Gln Cys Pro Lys Ala
   65                  70                  75                  80
   Glu Lys Met Ala Gln Asp Lys Leu Arg Ala Glu Glu Leu Ala Lys Glu
                   85                  90                  95
   Lys Ile Glu Ala Glu Lys Glu Pro Xaa Lys Pro
               100                 105
<210> 19
   <211> 464
   <212> DNA
   <213> Parthenium argentatum Grey
<220>
   <221> unsure
   <222> (445)
<220>
   <221> unsure
   <222> (452)
<400> 19
   caagtgttct tgagcttcag tgcattgatc caaaatggcc aaaacttcag ttgctttctt  60
   tgcatttctt ctgctcctcg ttcttgctat ctcagaaatc ggatcggtga agggagaact 120
   atgtgagaag gcaagcaaga catggtctgg aaattgtggt aacacaagac actgtgacga 180
   ccaatgcaag tcttgggagg gtgcagccca tggagcttgt catgtgcgcg gtgggaaaca 240
   catgtgcttc tgctacttcc agtgccccaa agccgagaag atggcccagg ataaactccg 300
   agctgaagag cttgccaagg agaagattga agttgaaaaa gagccaacca aaccttgagt 360
   agcaaatgtt atgtttatga ataaagagaa aatgctttct acttacatgt tcaacaattt 420
   ccaaggggga aatgtttccc tgcanttgga ancaattgtt atga                 464
<210> 20
   <211> 107
   <212> PRT
   <213> Parthenium argentatum Grey
<400> 20
   Met Ala Lys Thr Ser Val Ala Phe Phe Ala Phe Leu Leu Leu Leu Val
   1               5                   10                  15
   Leu Ala Ile Ser Glu Ile Gly Ser Val Lys Gly Glu Leu Cys Glu Lys
               20                  25                  30
   Ala Ser Lys Thr Trp Ser Gly Asn Cys Gly Asn Thr Arg His Cys Asp
           35                  40                  45
   Asp Gln Cys Lys Ser Trp Glu Gly Ala Ala His Gly Ala Cys His Val
       50                  55                  60
   Arg Gly Gly Lys His Met Cys Phe Cys Tyr Phe Gln Cys Pro Lys Ala
   65                  70                  75                  80
   Glu Lys Met Ala Gln Asp Lys Leu Arg Ala Glu Glu Leu Ala Lys Glu
                   85                  90                  95
   Lys Ile Glu Val Glu Lys Glu Pro Thr Lys Pro
               100                 105
<210> 21
   <211> 458
   <212> DNA
   <213> Parthenium argentatum Grey
<220>
   <221> unsure
   <222> (322)
<220>
   <221> unsure
   <222> (375)
<220>
   <221> unsure
   <222> (402)
<220>
   <221> unsure
   <222> (452)
<400> 21
   cattgatcca aaatggccaa aacttcagtt gctttctttg catttcttct gctcctcgtt  60
   cttgctatct cagaaatcgg atcggtgaag ggagaactat gtgagaaggc aagcaagaca 120
   tggtctggaa attgtggtaa cacaagacac tgtgacgacc aatgcaagtc ttgggagggt 180
   gcagcccatg gagcttgtca tgtgcgcggt gggaaacaca tgtgcttctg ctacttccag 240
   tgccccaaag ccgagaagat ggcccaggat aaactccgag ctgaagagct tgccaaggag 300
   aagattgaag ctgaaaaaga gncaagccaa accttgagta gcaaaatgtt atgcttatga 360
   ataagagaaa atgcnttcta cttaaatctt taacaatttt cnaaggggta atgtttcctg 420
   catttggaaa caattgttat gattcccttg tnaaaagg                        458
<210> 22
   <211> 115
   <212> PRT
   <213> Parthenium argentatum Grey
<220>
   <221> UNSURE
   <222> (104)
<400> 22
   Met Ala Lys Thr Ser Val Ala Phe Phe Ala Phe Leu Leu Leu Leu Val
   1               5                   10                  15
   Leu Ala Ile Ser Glu Ile Gly Ser Val Lys Gly Glu Leu Cys Glu Lys
               20                  25                  30
   Ala Ser Lys Thr Trp Ser Gly Asn Cys Gly Asn Thr Arg His Cys Asp
           35                  40                  45
   Asp Gln Cys Lys Ser Trp Glu Gly Ala Ala His Gly Ala Cys His Val
       50                  55                  60
   Arg Gly Gly Lys His Met Cys Phe Cys Tyr Phe Gln Cys Pro Lys Ala
   65                  70                  75                  80
   Glu Lys Met Ala Gln Asp Lys Leu Arg Ala Glu Glu Leu Ala Lys Glu
                   85                  90                  95
   Lys Ile Glu Ala Glu Lys Glu Xaa Ser Gln Thr Leu Ser Ser Lys Met
               100                 105                 110
   Leu Cys Leu
           115
<210> 23
   <211> 472
   <212> DNA
   <213> Parthenium argentatum Grey
<220>
   <221> unsure
   <222> (368)
<400> 23
   cttgagcttc agtgcattga tccaaaatgg ccaaaacttc agttgctttc tttgcatttc  60
   ttctgctcct cgttcttgct atctcagaaa tcggatcggt gaagggagaa ctatgtgaga 120
   aggcaagcaa gacatggtct ggaaattgtg gtaacacaag acactgtgac gaccaatgca 180
   agtcttggga gggtgcagcc catggagctt gtcatgtgcg cggtgggaaa cacatgtgct 240
   tctgctactt ccagtgcccc aaagccgaga agatggccca ggataaactc cgagctgaag 300
   agcttgccaa ggagaagatt gaagttgaaa aagagccagc caaaccttga gtagcaaatg 360
   ttatgttnat gaataaagag aaaatgcttt ctacttacat gttcaacatt ttctatgggg 420
   taatgtttct tgcatttggg aatcaattgt tatgattcct tggtaaaatg tt         472
<210> 24
   <211> 107
   <212> PRT
   <213> Parthenium argentatum Grey
<400> 24
   Met Ala Lys Thr Ser Val Ala Phe Phe Ala Phe Leu Leu Leu Leu Val
   1               5                   10                  15
   Leu Ala Ile Ser Glu Ile Gly Ser Val Lys Gly Glu Leu Cys Glu Lys
               20                  25                  30
   Ala Ser Lys Thr Trp Ser Gly Asn Cys Gly Asn Thr Arg His Cys Asp
           35                  40                  45
   Asp Gln Cys Lys Ser Trp Glu Gly Ala Ala His Gly Ala Cys His Val
       50                  55                  60
   Arg Gly Gly Lys His Met Cys Phe Cys Tyr Phe Gln Cys Pro Lys Ala
   65                  70                  75                  80
   Glu Lys Met Ala Gln Asp Lys Leu Arg Ala Glu Glu Leu Ala Lys Glu
                   85                  90                  95
   Lys Ile Glu Val Glu Lys Glu Pro Ala Lys Pro
               100                 105
<210> 25
   <211> 499
   <212> DNA
   <213> Nicotiana benthamiana
<220>
   <221> unsure
   <222> (451)
<220>
   <221> unsure
   <222> (456)
<400> 25
   gagaagtagc ataaatttct aaatccatat tcatcatgag caacaaagtc tttctagcca  60
   tcttgttttg cttcctcctc attgcatcca atgagatgca aggaggagag gcgaaagttt 120
   gccaaaggcg aagcaagaca tggtcggggc cttgtattaa cacaggcaac tgcagccgtc 180
   aatgcaagaa tcaagaggat gctcgctttg gtgcttgtca cagaagtggg attggatttg 240
   cttgcttctg ctatttcaac tgctaaacga ccaaaaaaag accctatgtg ttttgtctct 300
   atttctatga ttgtactatc aaatatgtaa gtgttgtgtg tctgtatgaa taaagggcgt 360
   ccaatttact agaattggaa gtagccctag ttgtgttggt gtgtcattcc taaactttgt 420
   atttcaatct gggccagctt ttgttggtcc nattantaaa cgacgagtgc gtccacttgt 480
   aatcccccca aaaaaaaaa                                               499
<210> 26
   <211> 50
   <212> PRT
   <213> Nicotiana benthamiana
<400> 26
   Lys Val Cys Gln Arg Arg Ser Lys Thr Trp Ser Gly Pro Cys Ile Asn
   1               5                   10                  15
   Thr Gly Asn Cys Ser Arg Gln Cys Lys Asn Gln Glu Asp Ala Arg Phe
               20                  25                  30
   Gly Ala Cys His Arg Ser Gly Ile Gly Phe Ala Cys Phe Cys Tyr Phe
           35                  40                  45
   Asn Cys
       50
<210> 27
   <211> 517
   <212> DNA
   <213> Nicotiana benthamiana
<400> 27
   gcacgaggag aagtagcata aatttctaaa tccatattca tcatgagcaa caaagtcttt  60
   ctagccatct tgttttgctt cctcctcatt gcatccaatg agatgcaagg aggagaggcg 120
   aaagtttgcc aaaggcgaag caagacatgg tcggggcctt gtattaacac aggcaactgc 180
   agccgtcaat gcaagaatca agaggatgct cgctttggtg cttgtcacag aagtgggatt 240
   ggatttgctt gcttctgcta tttcaactgc taaacgacca aaaaaagacc ctatgtgttt 300
   tgtctctatt tctatgattg tactatcaaa tatgtaagtg ttgtgtgtct gtatgaataa 360
   agggcgtcca atttactaga attggaagta gccctagttg tgttggtgtg tcattcctaa 420
   actttgtatt tcaatctggc cagcttttgt tggtctatta ttaaacgacg agtgcgtcca 480
   cttgttattc cctccaaaaa aaaaaaaaaa aaaaaaa                          517
<210> 28
   <211> 76
   <212> PRT
   <213> Nicotiana benthamiana
<400> 28
   Met Ser Asn Lys Val Phe Leu Ala Ile Leu Phe Cys Phe Leu Leu Ile
   1               5                   10                  15
   Ala Ser Asn Glu Met Gln Gly Gly Glu Ala Lys Val Cys Gln Arg Arg
               20                  25                  30
   Ser Lys Thr Trp Ser Gly Pro Cys Ile Asn Thr Gly Asn Cys Ser Arg
           35                  40                  45
   Gln Cys Lys Asn Gln Glu Asp Ala Arg Phe Gly Ala Cys His Arg Ser
       50                  55                  60
   Gly Ile Gly Phe Ala Cys Phe Cys Tyr Phe Asn Cys
   65                  70                  75
<210> 29
   <211> 50
   <212> PRT
   <213> Dahlia merckii
<400> 29
   Glu Leu Cys Glu Lys Ala Ser Lys Thr Trp Ser Gly Asn Cys Gly Asn
   1               5                   10                  15
   Thr Gly His Cys Asp Asn Gln Cys Lys Ser Trp Glu Gly Ala Ala His
               20                  25                  30
   Gly Ala Cys His Val Arg Asn Gly Lys His Met Cys Phe Cys Tyr Phe
           35                  40                  45
   Asn Cys
       50